# EUROPÄISCHE PATENTANMELDUNG

(11) **EP 2 602 329 A1**
(43) Veröffentlichungstag der Anmeldung: **12.06.2013**
(21) Anmeldenummer: 11191923.9
(22) Anmeldetag: 05.12.2011
(51) Int. Cl.: C12P 7/42, C12N 15/52, C12N 9/02

(54) **Biotechnologische Herstellung von 3-Hydroxyisobuttersäure**

(71) Anmelder: Evonik Degussa GmbH, 45128 Essen (DE)
(72) Erfinder: Haas, Thomas Dr., 48161 Münster (DE); Schaffer, Steffen Dr., 45699 Herten (DE); Pötter, Markus Dr., 48149 Münster (DE); Wessel, Mirja Dr., 44799 Bochum (DE); Pfeffer, Jan Christoph Dr., 45355 Essen (DE); Gehring, Christian, 45770 Marl (DE); Kirchner, Nicole, 45657 Recklinghausen (DE); Wittmann, Eva-Maria, 83301 Traunreut (DE)

(57) **Zusammenfassung**

Die Erfindung betrifft ein Verfahren umfassend die Schritte a) Bereitstellen von Isobuttersäure, b) Kontaktieren von Isobuttersäure mit der Kombination aus Isobutyrat-Kinase und Phosphotransisobutyrylase und/oder Isobutyryl-Coenzym A-Synthetase/Ligase und/oder Isobutyrat-Coenzym A-Transferase, c) Kontaktieren des Produktes aus Schritt a) mit Isobutyryl-Coenzym A-Dehydrogenase, d) Kontaktieren des Produktes aus Schritt b) mit Methacrylyl-Coenzym A-Hydratase, und e) Hydrolyse des Produktes aus Schritt d) unter Bildung von 3-Hydroxyisobuttersäure, wobei wenigstens eines der Enzyme in Form einer Zelle verwendet wird, die eine ihrem Wildtyp gegenüber verringerte Aktivität einer 3-Hydroxyisobuttersäure-Dehydrogenase oder einer Variante davon umfasst, eine Zelle, die wenigstens ein Enzym aus der Gruppe umfassend Isobutyryl-Coenzym A-Synthetase/Ligase, Isobutyrat-Coenzym A-Transferase, Isobutyrat-Kinase, Phosphotransisobutyrylase, Isobutyryl-Coenzym A-Dehydrogenase, Methacrylyl-Coenzym A-Hydratase und 3-Hydroxisobutyryl-Coenzym A-Hydrolase und eine gegenüber ihrem Wildtyp verringerte Aktivität einer 3-Hydroxyisobuttersäure-Dehydrogenase oder einer Variante davon aufweist, wobei die Zelle bevorzugt zusätzlich eine Monooxygenase, noch bevorzugter eine Monooxygenase des AlkBGT-Typs oder eine Variante davon aufweist und die Verwendung einer solchen Zelle zur Herstellung von 3-Hydroxyisobuttersäure.

## Beschreibung

Die Erfindung betrifft ein Verfahren umfassend die Schritte a) Bereitstellen von Isobuttersäure, b) Kontaktieren von Isobuttersäure mit der Kombination aus Isobutyrat-Kinase und Phosphotransisobutyrylase und/oder Isobutyryl-Coenzym A-Synthetase/Ligase und/oder Isobutyrat-Coenzym A-Transferase, c) Kontaktieren des Produktes aus Schritt b) mit Isobutyryl-Coenzym A-Dehydrogenase, d) Kontaktieren des Produktes aus Schritt c) mit Methacrylyl-Coenzym A-Hydratase, und e) Hydrolyse des Produktes aus Schritt d) unter Bildung von 3-Hydroxyisobuttersäure, wobei wenigstens eines der Enzyme in Form einer Zelle bereitgestellt wird, die eine ihrem Wildtyp gegenüber verringerte Aktivität einer 3-Hydroxyisobuttersäure-Dehydrogenase oder einer Variante davon umfasst, eine Zelle, die wenigstens ein Enzym aus der Gruppe umfassend Isobutyryl-Coenzym A-Synthetase/Ligase, Isobutyrat-Coenzym A-Transferase, Isobutyrat-Kinase, Phosphotransisobutyrylase, Isobutyryl-Coenzym A-Dehydrogenase-, Methacrylyl-Coenzym A-Hydratase und 3-Hydroxisobutyryl-Coenzym A-Hydrolase und eine gegenüber ihrem Wildtyp verringerte Aktivität einer 3-Hydroxyisobuttersäure-Dehydrogenase oder einer Variante davon aufweist, wobei die Zelle bevorzugt zusätzlich eine Monooxygenase, noch bevorzugter eine Alkanhydroxylase des AlkBGT-Typs oder eine Variante davon aufweist und die Verwendung einer solchen Zelle zur Herstellung von 3-Hydroxyisobuttersäure.

Methacrylsäure stellt eine der bedeutendsten industriell hergestellten Chemikalien dar. In Form ihres monomeren Methylesters wird sie für die Herstellung von Polymethylmethacrylat als Polymerisationsedukt benötigt, das der Öffentlichkeit unter den Handelsnamen Plexiglas bekannt und für eine Vielzahl von Einsatzgebieten unentbehrlich ist. Beispiele für die Verwendung von Polymethacrylat umfassen die Zahnmedizin, wo es für Prothesen eingesetzt wird, die Automobiltechnik, in der es für Blinker- und Rückleuchtengläser verwandt wird, die Optik, insbesondere als Material für Kontaktlinsen und Brillenglas, das Bauwesen, wo es als Polymerbeton sowie als Zwei-Komponenten-Klebstoff eingesetzt wird, die Textilindustrie als Bestandteil von Polyacrylfasern und im Haushalt als Material für Gegenstände wie Schüsseln und Bestecke.

Herkömmlich wird Methacrylsäure ausgehend von fossilen Rohstoffen wie Öl hergestellt. Beispielsweise können Isobutylen und tertiäres Butanol zu Methacrylein umgesetzt werde, das in der Folge weiter zu Methacrylat oxidiert wird (William Bauer, Jr. "Methacrylic Acid and Derivatives" in Ullmann's Encyclopedia of Industrial Chemistry 2002, Wiley-VCH, Weinheim). Alternativ können Amidsulfate von Methacrylsäure, die ausgehend von entsprechenden 2-Hydroxynitrilen produziert werden, unter Entstehung von Methacrylsäure hydrolysiert werden. Die industrielle Produktion von Methacrylsäure unter Nutzung derartiger Verfahren hängt jedoch nicht nur von einer kontinuierlichen Zufuhr fossiler Edukte ab, sondern erfolgt unter Verbrauch erheblicher Mengen aggressiver, umweltschädlicher Chemikalien. So wird zur Produktion von 1 kg Methacrylsäure durch Hydrolyse von Amidsulfaten der Methacrylsäure 1,6 kg Schwefelsäure benötigt.

Um die Abhängigkeit von fossilen Rohstoffen als Energieträger und Edukte für industrielle Synthesen zu überwinden, werden derzeit vielfältige Anstrengungen übernommen, die darauf abzielen, industriell nachgefragte Feinchemikalien biotechnologisch auf der Basis nachwachsender Rohstoffe zu erzeugen. Im Falle der Methacrylsäure bzw. des Methacrylsäuremethylesters bietet sich eine biotechnologische Syntheseroute über 3-Hydroxyisobuttersäure an, die chemisch oder enzymatisch leicht unter Bildung von Methacrylsäure dehydratisiert werden kann (William Bauer, Jr. "Methacrylic Acid and Derivatives" in Ullmann's Encyclopedia of Industrial Chemistry 2002, Wiley-VCH, Weinheim). Der Stand der Technik lehrt die Herstellung von 3-Hydroxyisobuttersäure aus Isobuttersäure unter Verwendung der von Wildtypisolaten von Bakterien und Hefen (Hasegawa *et al.*, 1981; Hasegawa *et al.*, 1982, WO 2007/141208 A2 und WO 2008/119738 A1). Als Substrat für die Herstellung von 3-Hydroxyisobuttersäure durch geeignete Stämme, beispielsweise durch *Candida rugosa*, dient ausschließlich Isobuttersäure. Die oben genannten Patentanmeldungen, WO 2007/141208 A2 und WO 2008/119738 A1, beschreiben gentechnisch veränderte Zelle und Verfahren für die Herstellung von 3-Hydroxyisobuttersäure aus Kohlenhydraten, Glycerin, Kohlendioxid, Methanol, L-Vallin, L-Glutamat, CO, Synthesegas, Methan usw. sowie deren weitere chemische Umsetzung zu Methacrylsäure oder Methacrylsäureestern.

Die Herstellung von 3-Hydroxyisobuttersäure über die oben beschriebenen biotechnologischen Verfahren ist derzeit jedoch nicht wirtschaftlich. Ein wesentliches Hindernis dabei sind die hohen Rohstoffkosten für Isobuttersäure bei gleichzeitig teilweise geringer Ausbeute bei deren Umsetzung oder Verwendung von geeigneten Mikroorganismen.

Vor diesem Hintergrund liegt der vorliegenden Erfindung die Aufgabe zu Grunde, ein verbessertes Verfahren zur biotechnologischen Herstellung von 3-Hydroxyisobuttersäure zu entwickeln, das den im Stand der Technik beschriebenen Verfahren mit Hinblick auf Ausbeute, Reinheit und Ressourcenbedarf überlegen ist.

Weiterhin besteht die der vorliegenden Erfindung zu Grunde liegende Aufgabe darin, ein biotechnologisches Verfahren zur Herstellung von 3-Hydroxyisobuttersäure ausgehend von unsubstituierten, insbesondere nicht-heteroatomhaltigen Alkanen zu entwickeln.

Weiterhin liegt der vorliegenden Erfindung die Aufgabe zu Grunde, ein biotechnologisches Verfahren zur Gewinnung von 3-Hydroxyisobuttersäure ausgehend von nachwachsenden Rohstoffen und/oder ohne Einsatz von oder mit geringerem Einsatz von gesundheitsschädlichen Edukten, Zwischenstufen, Katalysatoren oder Nebenprodukten zu entwickeln.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Erfindungsgemäß wird die Aufgabe in ein einem ersten Aspekt gelöst durch ein Verfahren umfassend die folgenden Schritte:
a) Bereitstellen von Isobuttersäure,
b) Kontaktieren von Isobuttersäure mit
   der Kombination aus Isobutyrat-Kinase und Phosphotransisobutyrylase und/oder
   Isobutyryl-Coenzym A-Synthetase/Ligase und/oder
   Isobutyrat-Coenzym A-Transferase,
c) Kontaktieren des Produktes aus Schritt b) mit Isobutyryl-Coenzym A-Dehydrogenase,
d) Kontaktieren des Produktes aus Schritt c) mit Methacrylyl-Coenzym A-Hydratase, und
e) Hydrolyse des Produktes aus Schritt d) unter Bildung von 3-Hydroxyisobuttersäure,
wobei wenigstens eines der in den Schritten b), c) und d) verwendeten Enzyme aus der Gruppe umfassend Isobutyrat-Kinase, Phosphotransisobutyrylase, Isobutyryl-Coenzym A-Synthetase/Ligase und Isobutyrat-Coenzym A-Transferase, bevorzugt alle, Enzyme in Form einer Zelle verwendet wird, die eine ihrem Wildtyp gegenüber verringerte Aktivität einer 3-Hydroxyisobuttersäure-Dehydrogenase oder einer Variante davon aufweist.

In einer ersten Ausführungsform des ersten Aspektes wird die Isobuttersäure durch Kontaktieren von Isobutan mit einer Monooxygenase, bevorzugt einer Alkanhydroxylase, noch bevorzugter einer des AlkBGT-Typs oder einer Variante davon, gebildet.

In einer zweiten Ausführungsform des ersten Aspektes, die auch eine Ausführungsform der ersten Ausführungsform darstellt, wird die Hydrolyse in Schritt e) durch Kontaktieren des Produktes aus Schritt d) mit einer 3-Hydroxisobutyryl-Coenzym A-Hydrolase erreicht.

In einer dritten Ausführungsform des ersten Aspektes, die auch eine Ausführungsform der ersten bis zweiten Ausführungsform darstellt, weist die Zelle sowohl die Isobutyryl-Coenzym A-Dehydrogenase in Schritt c) als auch die Methacrylyl-Coenzym A-Hydratase in Schritt d) als auch
die Kombination aus Isobutyrat-Kinase und Phosphotransisobutyrylase und/oder
Isobutyryl-Coenzym A-Synthetase/Ligase und/oder
Isobutyrat-Coenzym A-Transferase
auf.

In einer vierten Ausführungsform des ersten Aspektes, die auch eine Ausführungsform der ersten bis dritten Ausführungsform darstellt, weist die Zelle zusätzlich eine Alkanhydroxylase, bevorzugt eine des AlkBGT-Typs oder eine Variante davon, auf.

In einer fünften Ausführungsform des ersten Aspektes, die auch eine Ausführungsform der ersten bis vierten Ausführungsform darstellt, handelt es sich bei der 3-Hydroxyisobuttersäure-Dehydrogenase um Yali0F02607g oder eine Variante davon.

Erfindungsgemäß wird die Aufgabe in einem zweiten Aspekt gelöst durch eine Zelle, die wenigstens ein Enzym aus der Gruppe umfassend Isobutyryl-Coenzym A-Synthetase/Ligase, Isobutyrat-Coenzym A-Transferase, Isobutyrat-Kinase, Phosphotransisobutyrylase, Isobutyryl-Coenzym A-Dehydrogenase, Methacrylyl-Coenzym A-Hydratase und 3-Hydroxisobutyryl-Coenzym A-Hydrolase und eine gegenüber ihrem Wildtyp verringerte Aktivität einer 3-Hydroxyisobuttersäure-Dehydrogenase oder einer Variante davon aufweist.

In einer ersten Ausführungsform des zweiten Aspektes weist die Zelle zusätzlich zu einer Isobutyryl-Coenzym A-Dehydrogenase und zusätzlich zu einer Methacrylyl-Coenzym A-Hydratase
die Kombination aus Isobutyrat-Kinase und Phosphotransisobutyrylase und/oder
Isobutyryl-Coenzym A-Synthetase/Ligase und/oder
Isobutyrat-Coenzym A-Transferase.
bevorzugt darüber hinaus eine 3-Hydroxyisobutyryl-CoA-Hydrolase, auf.
auf.

In einer zweiten Ausführungsform des zweiten Aspektes, die auch eine Ausführungsform der ersten Ausführungsform darstellt, umfasst die Zelle weiter eine Alkanhydroxylase, bevorzugt eine des AlkBGT-Typs oder eine Variante davon.

In einer dritten Ausführungsform des zweiten Aspektes, die auch eine Ausführungsform der ersten Ausführungsform darstellt, handelt es sich bei der 3-Hydroxyisobuttersäure-Dehydrogenase und Yali0F02607g (XP_504911) oder eine Variante davon.

In einem dritten Aspekt wird das der Erfindung zu Grunde liegende Problem gelöst durch die Verwendung der Zelle nach einem der Ansprüche 7 bis 10 zur Herstellung von 3-Hydroxyisobuttersäure.

In einer Ausführungsform des dritten Aspektes handelt es sich bei der 3-Hydroxyisobuttersäure-Dehydrogenase um Yali0F02607g oder eine Variante davon.

In einer weiteren Ausführungsform des ersten, zweiten oder dritten Aspektes handelt es sich bei der Zelle um eine bakterielle oder niedere eukaryontische Zelle.

In einer weiteren Ausführungsform des ersten, zweiten oder dritten Aspektes handelt es sich bei der Zelle um eine Hefezelle aus der Gruppe von Gattungen handelt, die *Yarrowia*, *Candida*, *Saccharomyces*, *Schizosaccharomyces* und *Pichia* umfasst und es sich bevorzugt um *Yarrowia lipolytica* handelt.

In einem vierten Aspekt wird das der Erfindung zu Grunde liegende Problem gelöst durch eine Reaktionsmischung umfassend die Zelle nach dem zweiten Aspekt sowie Isobutan oder Isobuttersäure.

Die Erfinder der vorliegenden Erfindung überraschend haben festgestellt, dass die Inaktivierung eines Gens, welches für ein als 3-Hydroxyisobuttersäure-Dehydrogenase identifiziertes Enzym kodiert, in einem Mikroorganismus zu einer erhöhten Ausbeute an 3-Hydroxyisobuttersäure führt.

Die Erfinder haben weiterhin überraschend gefunden, dass es möglich ist 3-Hydroxyisobuttersäure biotechnologisch ausgehend von Alkanedukten, insbesondere Isobutan, herzustellen.

Die Ausführung des erfindungsgemäßen Verfahrens erfordert zunächst das Bereitstellen von Isobuttersäure. Zunächst besteht die Möglichkeit, hierzu im Handel erhältliche Isobuttersäure einzusetzen, aus der der Fachmann leicht geeignete Lösungen herstellen kann. Weiterhin besteht die Möglichkeit, Isobuttersäure unter Verwendung von isolierten Enzymen oder ganzen Organismen mit geeigneter Katalysefähigkeit ausgehend von anderen Edukten herzustellen, beispielsweise durch Kultivierung eines Organismus, der natürlich Isobuttersäure herstellt. In einer bevorzugten Ausführungsform wird die Isobuttersäure durch das Kontaktieren von Isobutan oder einem anderen geeigneten Alkanvorläufer mit einer geeigneten Monooxygenase, bevorzugt Alkanhydroxylase hergestellt, bei der es sich in einer besonders bevorzugten Ausführungsform um eine Alkanhydroxylase des AlkBGT-Typs oder einer Variante davon handelt. In einer bevorzugten Ausführungsform wird unter dem Begriff "Alkanhydroxylase", wie hierin verwendet, eine Oxidoreduktase verstanden, die in der Lage ist, gesättigte Kohlenwasserstoffe, besonders Isobutan oder 3-Hydroxyisobutan, zur Carbonsäure zu oxidieren, bevorzugt an einem terminalen Kohlenstoffatom. Der Stand der Technik beschreibt eine Reihe von geeigneten Mikroorganismen und Enzymen. So beschreibt die US 1978-896476 die Oxidation von Alkanen und Cykloalkanen unter Verwendung verschiedener Mikroorganismen (u. a. *Methylomonas*, *Methylococcus*, *Methylosinus* und *Methylobacterium*) unter Bildung von Alkoholen und Ketonen. Derilanko et al. (Journal of Applied Biochemistry, 1983, 5 (1 - 2), 107 - 120) beschreiben 16 neue Bakterienstämme, die in der Lage sind, gasförmige Alkane mit einer Kettenlänge von C2 bis zu C4 zu den entsprechenden Methylketonen, sekundären und primären Alkoholen sowie Aldehyden zu oxidieren.

Andererseits besteht die Möglichkeit, Isobuttersäure durch geeignete Stämme von Mikroorganismen herzustellen, die natürlich oder durch genetische Modifikation mit Stoffwechselwegen ausgestattet sind, die die Herstellung von Isobuttersäure bei Fütterung mit geeigneten Kohlenstoffquellen, beispielsweise Glukose, umfassen. Beispielhafte Mikroorganismen umfassen z. B. *Yarrowia lipolytica*, *Candida rugosa*, *Hanseniaspora valbyensis*, *Hansenula anomala, Trichosporon aculeatum, Trichosporon fennicum, Endomyces reessii*, *Geotrichum loubieri, Micrococcus flavus, Micrococcus luteus, Micrococcus lysodeikticum, Candida parapsilosis, Pichia membranaefaciens, Torulopsis candida, Coccidioides posadasii, Coccidioides immitis, Verticillium dahliae, Gibberella* zeae, *Thielavia terrestris, Metarhizium acridum, Magnaporthe oryzae, Sordaria macrospora, Metarhizium nisopliae, Ajellomyces dermatitidis, Chaetomium globosum, Paracoccidioides brasiliensis, Nectria haematococca, Neurospora tetrasperma, Chaetomium thermophilum und Neurospora crassa.*

In einer bevorzugten Ausführungsform wird die Isobuttersäure durch Oxidation von Isobutan durch die Oxidoreduktase AlkB aus dem AlkBGT-System aus *Pseudomonas putida* oder einer Variante davon bereitgestellt. AlkB stellt eine Oxidoreduktase aus dem AlkBGT-System aus *Pseudomonas putida* dar, die für ihre Alkanhydroxylase-Aktivität bekannt ist. Diese ist von zwei weiteren Polypeptiden, AlkG und AlkT abhängig. AlkT wird als FAD-abhängige Rubredoxin-Reduktase charakterisiert, die Elektronen aus NADH an AlkG weitergibt. AlkG ist ein Rubredoxin, ein eisenhaltiges Redoxprotein, das als direkter Elektronendonor für AlkB fungiert. In einer bevorzugten Ausführungsform wird unter dem Begriff "Alkanhydrohylase des AlkBGT-Typs", wie hierein verwendet, eine membranständige Alkanmonooxidase verstanden. In einer weiteren bevorzugten Ausführungsform wird unter dem selben Begriff "Alkanhydrohylase des AlkBGT-Typs" ein Polypeptid mit einer Sequenzhomologie von zunehmend bevorzugt wenigstens 75, 80, 85, 90, 92, 94, 96, 98 oder 99 % zur Sequenz des AlkB von Pseudomonas *putida* Gpo1 (Datenbankcode: CAB54050.1) verstanden. In einer weiteren bevorzugten Ausführungsform wird unter dem Begriff eine Cytochrom-unabhängige Monooxygenase verstanden. In einer weiteren bevorzugten Ausführungsform wird unter dem Begriff ""Alkanhydrohylase des AlkBGT-Typs" eine Cytochrom-unabhängige Monooxygenase verstanden, die wenigstens ein Rubredoxin oder Homologon als Elektronendonor verwendet. In einer besonders bevorzugten Ausführungsform wird unter dem Begriff eine membranständige, Cytochrom-unabhänige Alkanmonooxygenase mit zunehmend bevorzugt wenigstens 60, 70, 80, 80, 85, 90, 92, 94, 96, 98 oder 99 % zur Sequenz des AlkB von Pseudomonas *putida* Gpo1 verstanden, die als Elektronendonor wenigstens AlkG (CAB54052.1), bevorzugt aber die Kombination von AlkG mit der Reduktase AlkT (CAB54063.1) benötigt, wobei es sich bei alkG und/oder alkT auch um ein Homologon des jeweiligen Polypeptides handeln kann. Der Begriff "Sequenz", wie hierin verwendet, kann sich auf die Aminosäuresequenz eines Polypeptides und/oder die dafür codierende Nukleinsäuresequenz beziehen. In einer weiteren bevorzugten Ausführungsform handelt es sich bei einer "Oxidoreduktase des alkB-Typs", wie hierin verwendet, um eine Cytochrom-unabhängige Oxidoreduktase, d.h. eine Oxidoreduktase, die nicht Cytochrom als Cofaktor umfasst.

Es besteht die Möglichkeit, zu diesem Zweck gereinigte Komponenten des AlkBGT-Systems mit Isobutan zu kontaktieren, insbesondere AlkB. In einer bevorzugten Ausführungsform wird Isobutan mit einem AlkBGT-haltigen Ganzzellkatalysator kontaktiert, in einer bevorzugtesten Ausführungsform mit einem rekombinanten *E.coli*-Stamm der heterologes AlkBGT exprimiert.

Die Lehre der vorliegenden Erfindung kann nicht nur unter Verwendung der exakten Aminosäure- oder Nukleinsäuresequenzen der hierin beschriebenen biologischen Makromoleküle ausgeführt werden, sondern auch unter Verwendung von Varianten derartiger Makromoleküle, die durch Deletion, Addition oder Substitution einer oder mehr als einer Aminosäuren oder Nukleinsäuren erhalten werden können. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -Aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder letztere lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3^{rd} edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatische Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}- Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Alkoholdehydrogenase, Monooxygenase oder Transaminase. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu anellieren, die in ihrer Umgebung vorhanden sind, und zwar unterhalb der Schmelztemperatur. Die Stringenz von Hybridisierungsreaktion und entsprechende Bedingungen sind ausführlicher in Ausubel *et al.* 1995 beschrieben. In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Nach dem Bereitstellen von Isobuttersäure wird diese erfindungsgemäß mit einem Enzym oder Enzym-System kontaktiert, das in der Lage ist, sie zu Isobutyryl-CoA umzuwandeln. Möglich ist das Kontaktieren von Isobuttersäure mit der Kombination aus Isobutyrat-Kinase und Phosphortransisobutyrylase. Unter dem Begriff "Isobutyrat-Kinase", wie hierin verwendet, wird dabei ein Enzym verstanden, das in der Lage ist, Isobuttersäure unter Hydrolyse von ATP zu phosphorylieren. In einer besonders bevorzugten Ausführungsform wird unter dem Begriff "Phosphotransisobutyrylase", wie hierin verwendet, ein Enzym verstanden, das die Umsetzung der phosphorylierten Buttersäure unter Verbrauch von Coenzym A zu Isobutyryl-CoA zu katalysieren. Geeignete Enzyme kann der Fachmann dem Stand der Technik entnehmen beispielsweise: NP_348286.1, YP_001311072.1, YP_001311673.1, YP_003845108.1, CCC57671.1, ZP_02993103.1, YP_001255907.1, YP_001788766.1, YP_001783065.1, ZP_02613551.1, ZP_05129586.1, NP_783068.1, ZP_02616584.1, YP_001392779.1, ZP_02642313.1, YP_697036.1, NP_563263.1, YP_699607.1, ZP_05129585.1, ZP_05394270.1, ZP_04821992.1, YP_001086582.1, YP_001884532.1, YP_001919732.1, YP_001513941.1, YP_001322041.1, NP_349675.1, YP_001307350.1, ZP_02074622.1, AAA75487.1, ZP_05979314.1, ZP_02950703.1, YP_003935519.1, YP_001126403.1, ZP_03147541.1, BAD11094.1, ZP_08532470.1, CBK83142.1, ZP_02027123.1, ZP_02206646.1, ZP_03226899.1, ZP_05791023.1, YP_002950277.1, YP_003825217.1, ZP_08609344.1, YP_004819378.1, YP_001376086.1, ZP_06425397.1, ZP_04152833.1, ZP_03292064.1, ZP_07525976.1, YP_003477715.1, YP_001664465.1, ZP_04218902.1, YP_003677577.1, ZP_02211576.1, ZP_04187800.1, ZP_04302360.1, ZP_04098303.1, ZP_00394509.1, YP_085496.1, NP_846616.1, ZP_04313571.1, YP_896508.1, NP_980529.1, ZP_04170515.1, YP_001646798.1, ZP_00240356.1, ZP_04269443.1, ZP_04291055.1, ZP_04176201.1, ZP_01860365.1, ZP_03237173.1, YP_001471381.1, ZP_08211979.1, ZP_04208847.1, YP_004820818.1, ZP_04103879.1, YP_003666354.1, NP_833876.1, ZP_04285820.1, ZP_04086212.1, ZP_04147476.1, NP_623752.1, ZP_03231885.1, ZP_04066826.1, ZP_08211238.1, ZP_07709086.1, YP_002315321.1, YP_002447739.1, ZP_00741048.1, ZP_04073820.1, YP_003988617.1, YP_002368967.1, YP_148233.1, YP_003251443.1, AEN87678.1, YP_003564885.1, YP_004660977.1, YP_004587379.1, NP_349675.1, AAA75487.1, YP_001788766.1, YP_001255907.1, ZP_02993103.1, YP_001783065.1, ZP_02613551.1, ZP_02616584.1, YP_001392779.1, ZP_05394270.1, NP_783068.1, CCC57671.1, YP_697036.1, NP_563263.1, YP_699607.1, ZP_02642313.1, YP_001919732.1, ZP_07525976.1, YP_001884532.1, ZP_04821992.1, ZP_03292064.1, ZP_05129586.1, YP_001307350.1, ZP_02950703.1, ZP_05129585.1, YP_001086582.1, YP_001311072.1, YP_001513941.1, YP_003845108.1, YP_001322041.1, ZP_06425397.1, YP_001311673.1, YP_003935519.1, NP_348286.1, ZP_02027123.1, ZP_08532470.1, YP_001664465.1, YP_002950277.1, YP_004820818.1, ZP_07709086.1, ZP_08211979.1, ZP_02211576.1, ZP_05979314.1, ZP_02074622.1, YP_003677577.1, CBK83142.1, YP_003477715.1, ZP_02206646.1, BAD11094.1, ZP_01860365.1, ZP_03226899.1, NP_623752.1, YP_003988617.1, ZP_08005605.1, YP_003599607.1, YP_003831638.1, YP_003564885.1, YP_004587379.1, AEN87678.1, YP_001376086.1, YP_004819378.1, ZP_04152833.1, YP_001126403.1, ZP_03147541.1, ZP_04218902.1, ZP_08609344.1, ZP_00240356.1, YP_002368967.1, ZP_04147476.1, ZP_04176201.1, ZP_04269443.1, YP_003666354.1, ZP_04073820.1, ZP_04103879.1, NP_833876.1, ZP_04285820.1, ZP_04187800.1, ZP_04313571.1, ZP_03231885.1, ZP_03237173.1, ZP_04086212.1, ZP_04302360.1, ZP_08094177.1, NP_980529.1, ZP_04098303.1, YP_085496.1, ZP_04170515.1, YP_002447739.1, BAD11089.1, ZP_00394509.1, YP_001646798.1, YP_002315321.1, NP_846616.1, ZP_04066826.1, YP_896508.1, YP_003866727.1, ZP_00741048.1, YP_001487375.1, ZP_06875892.1, ZP_04291055.1

Sämtliche in dieser Anmeldung für die Angabe von Sequenzen aus dem Stand der Technik verwendete Datenbankencodes stammen aus der Datenbank NCBI (National Center for Biotechnology Information, Zugriffsdatum: 19.10.2011), genauer unter Verwendung des am 19.10.2011 online verfügbaren Releases.

Alternativ kann Isobutyryl-CoA unter Verwendung von Isobutyryl-Coenzym A-Synthetase oder -Ligase aus Isobuttersäure erhalten werden. In einer bevorzugten Ausführungsform wird unter dem Begriff "Isobutyryl-Coenzym A-Ligase", wie hierin verwendet, ein Enzym verstanden, das die Umwandlung von Isobuttersäure zu Isobutyryl-CoA unter Verbrauch von Coenzym A und Nucleotid-Triphosphat katalysiert. In einer besonders bevorzugten Ausführungsform wird unter dem Begriff "Isobutyryl-Coenzym A-Synthetase", wie hierin verwendet, eine Isobutyryl-Coenzym A-Ligase verstanden, wobei es sich bei dem im Zuge der Reaktion hydrolysierten NTP um ATP handelt. Geeignete Enzyme kann der Fachmann dem Stand der Technik entnehmen beispielsweise: NP_579516.1, NP_125992.1, YP_004423263.1, YP_004070968.1, YP_182878.1, YP_002306709.1, YP_002959654.1, YP_004762301.1, YP_002581616.1, YP_002994502.1, YP_004623157.1, NP_143577.1, YP_002307387.1, NP_579566.1, YP_004623106.1, YP_004763660.1, YP_002583149.1, YP_002959108.1, YP_002993622.1, YP_001736558.1, YP_003649375.1, YP_004423314.1, ZP_04874839.1, YP_183356.1, ZP_04874991.1, YP_001041242.1, YP_003860266.1, NP_126044.1, NP_143628.1, YP_003669211.1, YP_002428622.1, YP_001013511.1, YP_004175933.1, YP_004174284.1, YP_001012369.1, YP_004781520.1, YP_004071372.1, NP_769799.1, YP_429257.1, CAJ70793.1, ZP_08257475.1, YP_930006.1, NP_618478.1, ZP_08667208.1, YP_001239140.1, YP_004438172.1, YP_003400029.1, YP_001206872.1, NP_070039.1, YP_003727548.1, YP_004625200.1, ZP_08422040.1, YP_460839.1, ZP_08631067.1, ZP_02883796.1, YP_002953381.1, ZP_08110577.1, YP_003542795.1, ZP_08905433.1, ZP_06908198.1, YP_001581540.1, NP_632517.1, YP_004519194.1, YP_004515899.1, YP_004120624.1, ZP_07293826.1, CAJ73927.1, ZP_07331643.1, YP_001278815.1, ZP_08840735.1, YP_002289530.1, YP_001637486.1, YP_004342727.1, ZP_07026137.1, ZP_08112481.1, YP_003487360.1, YP_001540910.1, ZP_07946223.1, YP_685524.1, YP_004812635.1, ZP_08677213.1, ZP_08803651.1, ADI05837.1, YP_874976.1, YP_002465105.1, YP_003355503.1, ZP_07026765.1, YP_001430231.1, YP_004893707.1, YP_003766745.1, YP_004627663.1, YP_003649567.1, ZP_07307686.1, YP_001546514.1, YP_686303.1, YP_002992636.1, YP_004516207.1, YP_001154008.1, YP_004338243.1, YP_003357973.1

Schließlich kann Isobutyryl-CoA aus Isobuttersäure mittels Isobutyrat-Coenzym A-Transferase hergestellt werden. In einer bevorzugten Ausführungsform handelt es sich bei dem Begriff "Isobutyrat-Coenzym A-Transferase", wie hierin verwendet, um ein Enzym, das die Herstellung von Isobutyryl-CoA aus Isobuttersäure unter Transfer von Coenzym A von einem als Donor fungierenden Acyl-CoA katalysiert. Geeignete Enzyme kann der Fachmann dem Stand der Technik entnehmen beispielsweise: NP_149326.1, AAB53234.1, YP_001310904.1, AAD54947.1, AAP42564.1, CAQ57984.1, YP_001886322.1, NP_622378.1, ZP_08693244.1, ZP_07926619.1, ZP_08555875.1, ZP_04390377.1, ZP_01867058.1, ZP_04573915.1, ZP_07913714.1, ZP_07923474.1, ZP_08691337.1, ZP_08600063.1, ZP_02692961.1, ZP_08582386.1, ZP_00144733.1, ZP_05815087.1, ZP_06524353.1, ZP_07952599.1, YP_004254308.1, YP_003039857.1, YP_003828410.1, ZP_06175535.1, NP_602657.1, ZP_06748826.1, ZP_06749807.1, ZP_04970682.1, ADO77683.1, AAO18070.1, ZP_05887867.1, AEJ99145.1, EGB63075.1, NP_931005.1, YP_003968227.1, ZP_08327315.1, ZP_06025832.1, YP_003260518.1, YP_003016746.1, YP_001452140.1, EGW68380.1, ZP_02424926.1, ZP_03828051.1, EGB72667.1, EFW53769.1, ZP_03001766.1, ZP_05402063.1, YP_049390.1, ZP_08690265.1, YP_003936148.1, YP_002382110.1, NP_416725.1, YP_003941041.1, YP_001744415.1, YP_003296165.1, ZP_07151665.1, NP_754650.1, YP_002413271.1, EGC06740.1, YP_001459023.1, ZP_05272741.1, ZP_04005084.1, YP_541501.1, YP_002335226.1, YP_001089188.1, ZP_04054428.1, YP_001784143.1, YP_001306376.1, ZP_07820010.1, ZP_03065638.1, YP_004441693.1, NP_905281.1, YP_001321984.1, ZP_06636026.1, YP_003296060.1, ZP_07903700.1, YP_004509865.1, ZP_08626421.1, YP_002933615.1, YP_002771575.1, ZP_06715088.1, ZP_01548307.1, ZP_06982396.1, ZP_08643079.1, YP_002497558.1, YP_001513889.1, NP_438933.1, YP_248482.1, YP_531878.1, YP_002746872.1, YP_002744076.1, ZP_01792337.1, YP_002940319.1, YP_001471175.1, ZP_01220381.1, YP_002123783.1, NP_149327.1, CAQ57985.1, YP_001886321.1, AAD54948.1, YP_001310905.1, AAP42565.1, ZP_05092257.1, NP_622379.1, ZP_08555876.1, ZP_02692960.1, YP_002335225.1, YP_001306375.1, ZP_08693245.1, YP_001513888.1, YP_001321983.1, ZP_07926620.1, ZP_02424916.1, YP_001409735.1, ZP_06983458.1, YP_003828409.1, YP_004707898.1, YP_003936149.1, ZP_07577382.1, YP_001089189.1, YP_001471174.1, ZP_05272742.1, YP_002940318.1, ZP_05402064.1, YP_004254317.1, YP_003968226.1, CBK81879.1, ZP_07819217.1, YP_001740168.1, YP_049389.1, ZP_08709179.1, YP_001918401.1, YP_003016745.1, NP_905290.1, YP_004509856.1, ZP_03828050.1, YP_426559.1, YP_001929287.1, YP_001568118.1, ZP_04390271.1, ZP_07913715.1, ZP_07820042.1, YP_003260519.1, ZP_08691338.1, ZP_01548308.1, ZP_08327314.1, ZP_04970681.1, ZP_04054413.1, ZP_08690266.1, ZP_05887868.1, ZP_06748825.1, YP_001918068.1, ZP_08731713.1, NP_602656.1, ZP_06524354.1, ZP_01867057.1, ZP_04573916.1, ZP_05815086.1, ZP_06025831.1, ZP_00144734.1, ZP_08600062.1, YP_001784144.1, EGQ80092.1, ZP_06175534.1, ADO77682.1, ZP_01220382.1, YP_597732.1, YP_003945474.1, YP_004219013.1, YP_002562694.1, YP_001788168.1, YP_002285170.1, NP_268527.1, YP_001392125.1, CBZ04738.1, ZP_06921170.1, ZP_07461446.1, YP_531879.1, YP_002805353.1, YP_003452384.1, YP_001255346.1, YP_001385091.1, YP_878775.1, EGC06741.1, NP_663914.1, YP_059486.1, YP_002382109.1, YP_001884900.1, ZP_07307813.1, ZP_02621896.1, ZP_07903699.1, ZP_08729700.1, YP_001309762.1, YP_001768507.1, YP_602949.1, EGL48602.1, XP_501388.1, XP_002841479.1, XP_002481641.1, XP_002147496.1, XP_001879757.1, XP_661332.1, XP_003295652.1, XP_001936644.1, AAK40365.1, XP_001802255.1, XP_002383405.1, XP_001390976.1, XP_001212457.1, EGP83670.1, XP_001227675.1, XP_003176313.1, XP_001268294.1, XP_751117.1, EGF82407.1, XP_001258382.1, EFW13413.1, XP_003065510.1, EGD93452.1, EEH22314.1, XP_001247983.1, EGE09225.1, XP_003022194.1, CBX98353.1, XP_003014185.1, XP_002564448.1, XP_003239146.1, EGX51025.1, EGO01287.1, XP_002792936.1, EFQ30503.1, XP_001838038.1, XP_002847737.1, EGO55093.1, XP_957979.1, XP_001547410.1, EGR48848.1, XP_003038014.1, EER44122.1, EFY99528.1, EGC42434.1, EEH09119.1, EFY92418.1, EGS22244.1, XP_002629107.1, XP_002548743.1, XP_001904655.1, EFX03923.1, XP_716276.1, XP_002422356.1, XP_388121.1, XP_001586993.1, EGX96527.1, XP_368380.1, NP_595848.1, XP_003345762.1, XP 759662.1, XP_003001963.1, EGG06029.1, XP_002175297.1, XP_001386078.2, XP_003040746.1, XP_459426.2, EDK36000.2, CBQ73919.1, EGU85384.1, XP_001525161.1, XP_001731221.1, XP_002490853.1, XP_001486721.1

Der nächste Schritt bei der erfindungsgemäßen biotechnologischen Synthese von 3-Hydroxyisobuttersäure umfasst das Kontaktieren des Produktes aus Schritt b), dem Isobutyryl-CoA, mit einer Isobutyryl-Coenzym A-Dehydrogenase. In einer bevorzugten Ausführungsform wird unter dem Begriff "Isobutyryl-Coenzym A-Dehydrogenase", wie hierin verwendet, ein Enzym verstanden, das die Oxidation von Isobutyryl-Coenzym A zu Methacrylyl-Coenzym A unter Freisetzung von Reduktionsäquivalenten katalysiert. Geeignete Beispiele umfassen die aus dem Stand der Technik bekannten Polypeptide mit den Datenbankcodes XP_501919.2, EDP50227.1, XP_001267173.1, XP_751977.1, EFW17827.1, XP_001241675.1, XP_003070631.1, XP_002376988.1, EGS22147.1, XP_001271742.1, XP_002794645.1, XP_001214528.1, XP_959931.1, EGO55678.1, EEH46977.1, XP_002543210.1, XP_001401697.1, EEH07104.1, BAE59223.1, EGU83504.1, XP_002627767.1, XP_002483661.1, EFX03379.1, XP_003048205.1, EGX54111.1, XP_002150528.1, EFQ35634.1, XP_001548029.1, XP_659303.1, XP_002841387.1, XP_001791413.1, XP_001904778.1, XP_390966.1, XP_003015640.1, EGD94205.1, XP_003233609.1, XP_003018507.1, XP_002561081.1, XP_360875.1, EFY92504.1, XP_003344493.1, CBX95087.1, EGC41158.1, EGX92432.1, XP_003169516.1, EFZ00003.1, EGR49157.1, EGP87134.1, XP_002849756.1, XP_003302688.1, XP_001937377.1, EEH 18077.1, XP_001227538.1, XP_001586947.1, AAK63186.1, XP_001538624.1, AAQ04622.1, EGF84480.1, EER45206.1, XP_502873.1, EGS21840.1, XP_752854.1, XP_003170973.1, CBF88712.1, XP 001264273.1, XP_003232243.1, EGD97329.1, XP_002479178.1, XP_658428.1, XP_002844533.1, EGE05996.1, XP_001223344.1, EGX95352.1, XP_001389698.2, CAK37343.1, EGR51332.1, XP_003068884.1, XP_002146883.1,
XP_001243830.1, CAP65331.1, XP_001268770.1, XP_001818195.1, EFQ34732.1, XP_001210950.1, XP_962250.1, EEH50726.1, XP_001804465.1, XP_003023750.1, EGO58886.1, CBX97678.1, XP_002561648.1, EEH15844.1, XP_389837.1, XP_363106.2, EFY97392.1, EFY90016.1, EGU81915.1, XP_002621381.1, EEQ89840.1, EGX53945.1, XP_003295397.1, XP_003011911.1, EER38173.1, XP_002792725.1, XP_003045885.1, XP_001935173.1, EGP88451.1, XP_001584930.1, XP_001836878.1, EGG04035.1, XP_003005525.1, XP_001553916.1, XP_762332.1, CBQ71452.1, EFX05387.1, XP_568632.1, XP_001884381.1, EGO01481.1, XP_003332014.1, XP_003197572.1, XP_001731213.1, XP_003026264.1, EGF80206.1, XP_003346492.1, XP_003324899.1, EEH09831.1, XP_002582344.1, EGT41105.1, XP_002640134.1, CBJ32167.1, XP_002607968.1, EFW43327.1, XP_003099748.1, CAF95757.1, NP_491859.1, XP_002471089.1, YP_001611803.1, XP_002904727.1, EGG17601.1, EFX71478.1, CBN81547.1, XP_002640162.1, ADY46184.1, XP_003099703.1, EFA76871.1, AAH82665.1, 2JIF_A, XP_001658431.1, CAD38535.2, AAH13756.1, NP_001124722.1, NP_001600.1, XP_003255101.1, XP_003283361.1, CAJ81939.1, XP_002640145.1, NP_491871.1, XP_001104844.1, AAH54428.1

Der nächste Schritt des erfindungsgemäßen Verfahrens umfasst die Hydratisierung von Methacrylyl-Coenzym A unter Bildung von 3-Hydroxyisobutyryl-CoA mittels eines Methacrylyl-Coenzym A-Hydratase. In einer bevorzugten Ausführungsform wird unter dem Begriff "Methacrylyl-Coenzym A-Hydratase", wie hierin verwendet, ein Enzym verstanden, das die Anlagerung eines Wassermoleküls an Methacrylyl-Coenzym A unter Bildung von 3-Hydroxyisobuttersäure katalysiert. Beispiele umfassen die aus dem Stand der Technik bekannten Polypeptide mit den Datenbankencodes XP_502475.1, XP_003067220.1, XP_001239658.1, XP_002567879.1, XP_002145078.1, XP_001259415.1, CAK97202.1, XP_001211164.1, XP_001401252.2, XP_753374.1, XP_664448.1, CBF71576.1, XP_001274572.1, XP_002340305.1, XP_002845201.1, XP_001824127.1, XP_002795848.1, XP_003304302.1, EGC44435.1, EER38804.1, EEH09966.1, XP_001936195.1, EEH50797.1, EEH15784.1, EGO61149.1, XP_003170409.1, XP_961123.1, EGS18121.1, XP_001554659.1, XP_003230996.1, EGE07573.1, XP_002621253.1, EGE81015.1, EEQ89969.1, XP_361538.2, XP_001224889.1, XP_002381216.1, CBX96718.1, EFY95366.1, XP_001595164.1, XP_001911969.1, EGX51365.1, EFY87978.1, EFQ29854.1, EGU81865.1, XP_003050469.1, XP_003352189.1, XP_002839588.1, XP_003002061.1, EGD96635.1, XP_003022976.1, XP_003010670.1, EFX03685.1, EGR47862.1, XP_002584325.1, XP_387195.1, EGX94938.1, XP_002616791.1, XP_462069.2, XP_001800531.1

Schließlich ist zur Freisetzung des gewünschten Produktes 3-Hydroxyisobuttersäure die Hydrolyse des Produktes aus Schritt d), dem 3-Hydroxyisobutyryl-CoA, erforderlich. Hierzu besteht zunächst die Möglichkeit, das Produkt durch Zugabe von Säure oder Base extremen pH-Bedingungen auszusetzen, die die Hydrolyse ohne Einwirkung eines weiteren Enzyms begünstigen. In einer besonders bevorzugten Ausführungsform erfolgt die Hydrolyse jedoch durch Kontaktieren des Produktes aus Schritt c) mit einer 3-Hydroxyisobutyryl-Coenzym A-Hydrolase. In einer bevorzugten Ausführungsform wird unter dem Begriff "3-Hydroxyisobutyryl-Coenzym A-Hydrolase", wie hierin verwendet, ein Enzym verstanden, das 3-Hydroxyisobutyryl-Coenzym A zu 3-Hydroxyisobuttersäure und Coenzym A hydrolysiert. Als Beispiele können hier folgende Proteine genannt werden: XP_504911.1, XP 003066853.1, XP_001246264.1, XP_385460.1, EFY92465.1, XP_363761.1, XP_003346508.1, EFY97405.1, EEQ90955.1, XP_002623105.1, XP_001223375.1, XP_002794385.1, XP_003046454.1, EGO58901. EEH47368.1, EGS21819.1, EEH 18429.1, AAK07843.1, XP_002540650.1, XP_002484211.1, XP_002150057.1, EFQ36202.1, EDP49675.1, XP_750988.1, XP_003235672.1, EGR51338.1, XP_003013762.1, XP_003019423.1, EGE05569.1, XP_003169276.1, EGD98503.1, EEH06901.1, XP_001544047.1, EGC47721.1, XP_001821058.1, EGE82172.1, XP_002842837.1, XP_002835633.1, EGX53716.1, EFX05960.1, XP_001590627.1, XP_001552254.1, XP_962266.2, EGX95378.1, XP_001392906.1, XP_003302932.1, CBF89164.1, EGU77782.1, EGP84417.1, XP_001258223.1, XP_001268201.1, XP_002569077.1, XP_001214240.1, EER38573.1, ADD19825.1, XP_001939164.1, XP_658197.1, XP_001248657.1, XP_315590.3, XP_003047713.1, EFR21351.1, EGP83804.1, EFX82586.1, EFN83706.1, XP_001799030.1, XP_002423077.1, XP_002073789.1, XP_001987737.1, XP_002050275.1, XP_002091863.1, XP_002005175.1, XP_001974736.1, EGF81169.1, EFW47689.1, XP_002149358.1, XP_002149354.1, CBY01415.1, NP_611373.1, CAP65353.1, XP_002569208.1, AAL39202.2, XP_002543763.1, XP_001664110.1, XP_002034506.1, XP_002565243.1, XP_003049329.1, XP_001360407.1, XP_001865614.1 Erfindungsgemäß werden in Schritt b) bis d) biologisch aktive Enzyme verwendet. Dabei kann es sich, solange wenigstens eines der Enzyme in Form einer Zelle verwendet wird, die eine ihrem Wildtyp gegenüber verringerte Aktivität einer 3-Hydroxyisobuttersäure-Dehydrogenase oder einer Variante davon aufweist, wie bei allen erfindungsgemäß eingesetzten enzymatisch aktiven Polypeptiden um Zellen umfassend enzymatisch aktive Polypeptide oder deren Lysate oder Präparationen der Polypeptide in sämtlichen Aufreinigungsstufen, von der intakten Zelle oder ihrem kruden Lysat bis zum reinen Polypeptid, handeln, die das jeweilige biologisch aktive Enzym in endogener oder rekombinanter Form aufweisen, bevorzugt überexprimiert. Dem Fachmann auf dem Gebiet sind zahlreiche Verfahren bekannt, mit denen enzymatisch aktive Polypeptide in geeigneten Zellen überexprimiert und aufgereinigt bzw. isoliert werden können. So können zur Expression der Polypeptide sämtliche dem Fachmann zur Verfügung stehende Expressionssysteme verwendet werden, beispielsweise Vektoren vom Typ pET oder pGEX. Zur Aufreinigung kommen chromatographische Verfahren in Frage, beispielsweise die affinitätschromatographische Aufreinigung eines mit einem Tag versehenen rekombinanten Proteins unter Verwendungen eines immobilisierten Liganden, beispielsweise eines Nickelions im Falle eines Histidin-Tags, von immobilisiertem Glutathion im Falle einer an das Zielprotein fusionierten Glutathion-S-Transferase oder von immobilisierter Maltose im Falle eines Tags umfassend Maltose-bindendes Protein. Dem Fachmann sind weiterhin bekannt, wie er im Rahmen routinemäßigen Experimentierens geeignete Reaktionsbedingungen herausarbeiten kann, unter denen das interessierende Enzym Aktivität, bevorzugt optimale Aktivität, zeigt. Diese Bedingungen umfassen beispielsweise die Wahl geeigneter Puffer, die Ermittlung und Einstellung des optimalen pH-Wertes, einer bestimmten Salz- und einer bestimmten minimalen Proteinkonzentration, siehe beispielsweise Cornish-Bowden, 1995.

Werden für das erfindungsgemäße Verfahren aufgereinigte Enzyme und nicht intakte lebende Zellen eingesetzt, so können erstere entweder in löslicher Form oder immobilisiert eingesetzt werden. Dem Fachmann sind geeignete Verfahren bekannt, mit denen Polypeptide an organischen oder anorganischen Festphasen kovalent oder nichtkovalent immobilisiert werden können, beispielsweise durch Sulfhydryl-Kopplungs-Chemie (z.B. Kits der Firma Pierce).

Da sich das erfindungsgemäße Verfahren jedoch mehrerer Enzyme mit unterschiedlichen Cofaktoren bedient, die im Falle aufgereinigter Polypeptide möglicherweise stöchiometrisch zugesetzt werden, werden die für das Verfahren erforderlichen Enzyme in einer besonders bevorzugten Ausführungsform in Form eines einzigen Ganzzellkatalysators bereitgestellt, d.h. in Form einer lebensfähigen, stoffwechselaktiven Zelle. Dabei können die Enzyme an der Oberfläche des Ganzzellkatalysators präsentiert werden, wie im Stand der Technik, beispielsweise in der DE 60216245 beschrieben ist. Ganz besonders bevorzugt ist jedoch, dass die von zu regenerienden Cofaktoren abhängigen Enzyme, noch bevorzugter alle Enzyme, derart lokalisiert sind, dass ihre aktiven Zentren mit dem Inneren der Zelle in Kontakt stehen, so dass die benötigten Cofaktoren und Cosubstrate aus dem Stoffwechsel der Zelle bezogen und wieder nachgeliefert werden.

Die Herstellung von Mutanten einer Zelle, die eine bestimmte enzymatische Aktivität aufweist, mit dem Ziel, diese enzymatische Aktivität bei der zu erhaltenen Mutante gegenüber dem Wildtyp der Zelle zu verringern, ist dem Fachmann unter Verwendung von Standardverfahren auf dem Gebiet der Molekularbiologie, der Genetik und der Mikrobiologie (Sambrook *et al.,* 1989) möglich. Beispielsweise ist eine ungerichtete Mutagenese durch Behandlung von Wildtyp-Zellen mit radioaktiver Strahlung gefolgt von einem Schritt zur Selektion geeigneter Mutanten durch Bestimmung der enzymatischen Aktivität isolierter Kolonien unter Verwendung von geeigneten Assays möglich, die für zahlreiche Enzyme im Stand der Technik beschrieben sind (Cornish-Bowden, 1995). Weitere Verfahren umfassend die Einfügung von deaktivierenden Punktmutationen, beispielsweise in den Promoter oder ins aktive Zentrum des enzymatisch aktiven Polypeptides, ein ebenfalls seit Jahrzehnten etabliertes Verfahren (Fersht and Winter, 2008).

In einer bevorzugten Ausführungsform handelt es sich bei der verwendeten Zelle um eine prokaryotische, bevorzugt um eine bakterielle Zelle. In einer weiteren bevorzugten Ausführungsform handelt es sich um eine Säugetierzelle. In einer weiteren bevorzugten Ausführungsform handelt es sich um eine niedere eukaryotische Zelle, bevorzugt um eine Hefezelle. Beispielhafte prokaryotische Zellen umfassen *Escherichia*, besonders *Escherichia coli*, und Stämme der Gattung *Pseudomonas* und *Corynebacterium*. Beispielhafte niedere eukaryotische Zellen umfassen die Gattungen *Saccharomyces*, *Candida*, *Pichia*, *Yarrowia*, *Schizosaccharomyces*, besonders die Stämme *Candida tropicalis*, *Schizosaccharomyces pombe*, *Pichia pastoris*, *Yarrowia lipolytica* und *Saccharomyces cerivisiae*. In der bevorzugtesten Ausführungsform handelt es sich um *Yarrowia lipolytica*.

Ein für die erfindungsgemäße Lehre wesentlicher Aspekt besteht darin, dass eine Zelle verwendet wird, deren 3-Hydroxyisobuttersäure-Dehydrogenase-Aktivität oder die Aktivität einer Varianten davon verringert ist. In einer bevorzugten Ausführungsform wird unter dem Begriff "3-Hydroxyisobuttersäure-Dehydrogenase-Aktivität", wie hierin verwendet, die Aktivität eines Enzyms verstanden, das 3-Hydroxyisobuttersäure zum Aldehyd oxidiert. In einer bevorzugten Ausführungsform handelt es sich bei der 3-Hydroisobuttersäure-Dehydrogenase um ein Enzyme aus der Gruppe, die die aus dem Stand der Technik bekannten Polypeptide XP_504911.1, XP_003066853.1, XP_001246264.1, XP_385460.1, EFY92465.1, XP_363761.1, XP_003346508.1, EFY97405.1, EEQ90955.1, XP_002623105.1, XP_001223375.1, XP_002794385.1, XP_003046454.1, EG058901.1, EEH47368.1, EGS21819.1, EEH 18429.1, AAK07843.1, XP_002540650.1, XP_002484211.1, XP_002150057.1, EFQ36202.1, EDP49675.1, XP_750988.1, XP_003235672.1, EGR51338.1, XP_003013762.1, XP_003019423.1, EGE05569.1, XP_003169276.1, EGD98503.1, EEH06901.1, XP_001544047.1, EGC47721.1, XP_001821058.1, EGE82172.1, XP_002842837.1, XP_002835633.1, EFX05960.1, XP_001590627.1, XP_001552254.1, XP_962266.2, XP_001392906.1, XP_003302932.1, CBF89164.1, EGP84417.1, XP_001258223.1, XP_001268201.1, XP_002569077.1, XP_001214240.1, EER38573.1, XP_001939164.1, XP_658197.1, XP_001248657.1, XP_003047713.1, EGP83804.1, XP_001799030.1, EGF81169.1, XP_002149358.1, XP_002149354.1, CBY01415.1, CAP65353.1, XP_002569208.1, XP_002543763.1, XP_002565243.1, XP_003049329.1, XP_001933034.1, XP_002551090.1, XP_001209304.1, XP_001796105.1, XP_003305815.1, CBY01417.1, XP_002144318.1, XP_003035025.1, XP_003047232.1, EDK39415.2, XP_003070936.1, XP_001878640.1, XP_001833463.1, XP_001484132.1, XP_003051032.1, XP_719244.1, XP_001796108.1, XP_456589.2, XP_001384410.2, XP_002421055.1, XP_719127.1, XP_001524095.1, XP_003336106.1, EGN96107.1, XP_003043164.1, XP_001903154.1, XP_758336.1, XP 003051445.1, CBX93804.1, XP_002614534.1, EFW22385.1, XP_003047715.1, XP_003009661.1, XP_001903159.1, XP_002144320.1, XP_754672.2, EGG05569.1 umfasst, noch bevorzugter um ein Enzyme aus der Gruppe, die XP_504911.1, XP_003066853.1, XP_001246264.1, XP_385460.1, EFY92465.1, XP_363761.1, XP_003346508.1, EFY97405.1, EEQ90955.1, XP_002623105.1, XP_001223375.1, XP_002794385.1, XP_003046454.1, EG058901.1, EEH47368.1, EGS21819.1, EEH 18429.1, AAK07843.1, XP_002540650.1, XP_002484211.1, XP_002150057.1, EFQ36202.1, EDP49675.1, XP_750988.1, XP_003235672.1, EGR51338.1, XP_003013762.1, XP_003019423.1, EGE05569.1, XP_003169276.1, EGD98503.1, EEH06901.1, XP_001544047.1, EGC47721.1, XP_001821058.1, EGE82172.1, XP_002842837.1, XP_002835633.1, EFX05960.1, XP_001590627.1, XP_001552254.1, XP_962266.2, XP_001392906.1, XP_003302932.1, CBF89164.1, EGP84417.1, XP_001258223.1, XP_001268201.1, XP_002569077.1, XP_001214240.1, EER38573.1, XP_001939164.1, XP_658197.1, XP_001248657.1, XP_003047713.1, XP_002551090.1, EDK39415.2, XP_001484132.1, XP_719244.1, XP_456589.2, XP_001384410.2, XP_002421055.1, XP_719127.1, XP_001524095.1, XP_002614534.1, XP_504911.1, besonders bevorzugt XP_504911.1 XP_002551090.1, EDK39415.2, XP_001484132.1, XP_719244.1, XP_456589.2, XP_001384410.2, XP_002421055.1, XP_719127.1, XP_001524095.1, XP_002614534.1, und am bevorzugtesten um XP_504911.1.

In einer bevorzugten Ausführungsform handelt es sich bei der 3-Hydroxyisobuttersäure-Dehydrogenase ume eine 3-Hydroxyisobuttersäure-Dehydrogenase aus der Gruppe, die die aus dem Stand der Technik bekannten Polypeptide BAC82381.1, NP_746775.1, YP_004703920.1, YP_001670886.1, ADR61938.1, YP_001269834.1, YP_001747642.1, YP_606441.1, BAJ07617.1, YP_257885.1, EGH62730.1, ZP_06461142.1, ZP_05642104.1, YP_004351842.1, EGH86869.1, YP_002874705.1, EGH24275.1, ZP_07777517.1, ZP_07003462.1, ZP_07264531.1, EGH72043.1, YP_233798.1, EGH66664.1, EGH33025.1, EGH77479.1, EGH54475.1, EFW86611.1, EGH13085.1, ZP_03399204.1, NP_790630.1, YP_346429.1, ZP_04590292.1, EGH46911.1, YP_004681354.1, YP_840711.1, YP_002007768.1, YP_298456.1, ZP_06495709.1, YP_001777122.1, YP_837675.1, YP_624179.1, YP_001117046.1, YP_555519.1, YP_004360017.1, ZP_07673810.1, YP_004714189.1, YP_003607711.1, YP_557465.1, YP_001810630.1, YP_003749513.1, ZP_08140457.1, AEA83842.1, YP_001861262.1, YP_001892508.1, ZP_03264187.1, ADY83615.1, YP_001844789.1, YP_002233766.1, ZP_02893156.1, ZP_06693498.1, YP_001705868.1, YP_001715485.1, YP_775329.1, ADX90568.1, ZP_06059137.1, ADX01770.1, ZP_05825497.1, ZP_06069662.1, YP_372791.1, YP_002254778.1, AEG71628.1, YP_001584514.1, ZP_02358230.1, ZP_03569839.1, YP_439994.1, ZP_02365286.1, YP_554218.1, YP_004473140.1, YP_001889364.1, ZP_03585594.1, YP_110641.1, YP_003734018.1, ZP_06839562.1, YP_001583635.1, ZP_00944342.1, ZP_03573494.1, YP_001115788.1, ZP_03582471.1, YP_776268.1, YP_623275.1, ZP_04522920.1, ZP_04947734.1, ZP_02910304.1, YP_371475.1, YP_001811564.1, YP_002798276.1, YP_003910785.1, YP_004230293.1, ZP_02881368.1, ZP_03822695.1, ZP_02891780.1, ZP_06726280.1, CBJ40252.1, ZP_02376988.1, ZP_05359923.1, YP_002908300.1, YP_004348870.1, YP_001172440.1, YP_003747923.1, YP_046276.1, NP_522210.1, YP_001156335.1, ZP_03268101.1, ZP_07236476.1, YP_001861075.1, ZP_05136769.1, YP_293155.1, YP_001083192.1, YP_001970187.1, YP_002026611.1, YP_003776462.1, ZP_02381313.1, ADP96674.1, EGF41785.1, ZP_08410855.1, ZP_05911432.1, AEL06416.1, ZP_01989307.1, ZP_06178628.1, NP_636638.1, ZP_01260019.1, ZP_04929809.1, ZP_08460617.1, YP_004068587.1, YP_002553266.1, ZP_06877402.1, YP_002439068.1, NP_800628.1, ZP_08100296.1, NP_252259.1, YP_001279202.1, ZP_01367000.1, YP_001346959.1, ZP_01133887.1, EGM15746.1, YP_339977.1, ZP_04921053.1, ZP_04764368.1, NP_762450.2, YP_986257.1, YP_789596.1, YP_004191179.1, YP_004416219.1, NP_937094.1, YP_002794225.1, ZP_02244049.1, ZP_05943914.1, YP_450771.1, YP_200485.1, ZP_08178115.1, NP_717293.1, ZP_06730192.1, ZP_04958952.1, ZP_03698449.1, ZP_04922804.1, YP_003288698.1, YP_001141739.1, ZP_08188278.1, YP_786279.1, ZP_05127790.1, YP_363098.1, ZP_01104144.1, ZP_05886382.1, ZP_05620602.1, ZP_01614260.1, NP_641651.1, ZP_06705559.1, ZP_01260775.1, ZP_06182137.1, ZP_05879122.1, YP_001631119.1, ZP_08552251.1, ZP_08570660.1, ZP_01738211.1, ZP_01893199.1, ZP_08520681.1, ZP_08182597.1, YP_264191.1, YP_001414164.1, ZP_08638362.1, YP_422950.1, EGF41343.1, ZP_00056040.1, ZP_08700476.1, NP_800135.1, ZP_08272093.1, ZP_08181910.1, ZP_02194300.1, ZP_03698989.1, YP_004235082.1 umfasst, bevorzugt um ein Enzym aus der Gruppe, die die Enzyme BAC82381.1, NP_746775.1, YP_004703920.1, YP_001670886.1, ADR61938.1, YP_001269834.1, YP_001747642.1, YP_606441.1, BAJ07617.1, YP_257885.1, EGH62730.1, ZP_06461142.1, ZP_05642104.1, YP_004351842.1, EGH86869.1, YP_002874705.1, EGH24275.1, ZP_07777517.1, ZP_07003462.1, ZP_07264531.1, EGH72043.1, YP_233798.1, EGH66664.1, EGH33025.1, EGH77479.1, EGH54475.1, EFW86611.1, EGH13085.1, ZP_03399204.1, NP_790630.1, YP_346429.1, ZP_04590292.1, EGH46911.1, ZP_06495709.1, YP_004714189.1, ZP_08140457.1, AEA83842.1, ADY83615.1, YP_001844789.1, ZP_06693498.1, YP_001705868.1, YP_001715485.1, ADX90568.1, ZP_06059137.1, ADX01770.1, ZP_05825497.1, ZP_06069662.1, YP_004473140.1, YP_003734018.1, YP_002798276.1, ZP_03822695.1, ZP_06726280.1, ZP_05359923.1, YP_001172440.1, YP_046276.1, ZP_07236476.1, YP_001083192.1, ZP_04929809.1, ZP_08460617.1, ZP_06877402.1, YP_002439068.1, NP_252259.1, YP_001279202.1, ZP_01367000.1, YP_001346959.1, EGM 15746.1, YP_789596.1, ZP_05620602.1, YP_264191.1, YP_580776.1, YP_792542.1, YP_004379211.1, ZP_06880384.1, YP_001186868.1, ZP_04932430.1, ZP_01364121.1, YP_002442182.1, NP_249434.1, YP_001350119.1, EGH98403.1, 3OBB_A, 3Q3C_A umfasst. In einer bevorzugten Ausführungsform bedeutet die Formulierung "eine Zelle, die eine gegenüber ihrem Wildtyp verringerte Aktivität einer 3-Hydroxyisobuttersäure-Dehydrogenase oder einer Variante davon aufweist", dass es sich um eine Zelle handelt, die ihrem Wildtyp gegenüber derartig gentechnisch verändert ist, dass die Aktivität einer 3-Hydroxyisobuttersäure-Dehydrogenase oder einer Variante bei dieser Zelle gegenüber der Aktivität der gleichen 3-Hydroyxisobuttersäure-Dehydrogenase oder der entsprechenden Variante beim Wildtyp der Zelle verringert ist, bevorzugt um wenigstens 10, 20, 30, 50, 75, 90, 95 oder 99 %. In einer besonders bevorzugten Ausführungsform ist die Aktivität des Enzyms bei der gentechnisch veränderten Zelle nicht mehr nachweisbar. In einer weiteren besonders bevorzugten Ausführungsform betrifft die gentechnische Modifikation, mit der die Aktivitätsverringerung bewerkstelligt wird, ausschließlich die Aktivität einer spezifischen 3-Hydroyxisobuttersäure-Dehydrogenase der Zelle, im Gegensatz zu der Möglichkeit, dass unspezifisch mehrere enzymatische Aktivitäten der Zelle verringert sind, beispielsweise durch einen Defekt in der Faltungsmaschinerie der Zelle, der bewirken könnte, dass zahlreiche enzymatische Aktivitäten der Zelle defekt sind. Ein Vergleich der enzymatischen Aktivitäten der gentechnisch veränderten Zelle und ihres Wildtyps erfolgt dabei unter gleichen Bedingungen und unter Verwendungen von Standardassays zur Bestimmung einer Dehydrogenaseaktivität. So lässt sich die Dehydrogenaseaktivität beispielsweise in einem kontinuierlichen spektrophotometrischen Assay verfolgen, wenn das Enzym, rein oder in Form eines Zellysates, mit Substrat, d.h. 3-Hydroxyisobuttersäure und Redoxfaktor inkubiert wird und der Reaktionsfortschritt anhand der Extinktion des Redoxfaktors verfolgt wird.

Zur Durchführung des erfindungsgemäßen Verfahrens kommen verschiedene Bedingungen in Frage. Die Temperatur kann dabei mit der Maßgabe, dass im Falle der Verwendung einer lebenden Zelle bzw. einer Präparation geeigneter Enzyme die gewählte Zelle bzw. die gewählten Enzyme lebensfähig sind bzw. Aktivität zeigen, mehr als 20 °C, 30 °C, 40, 50, 60, 70 °C oder mehr als 80 °C, bevorzugt bis 100 °C betragen. Dem Fachmann ist bekannt, welche Organismen bei welchen Temperaturen lebensfähig sind, beispielsweise aus Lehrbüchern wie Fuchs/Schlegel, 2007. Im Falle einer lebenden Hefezelle kann die Temperatur 5 bis 45 °C, bevorzugter 15 bis 42 °C, noch bevorzugter 20 bis 30 °C betragen. Im Falle eines Gramnegativen Bakteriums, bevorzugt eines Bakteriums aus der Familie der Enterobacteriaceae, am bevorzugtesten *E. coli,* kann die Temperatur 5 bis 45 °C, bevorzugter 15 bis 42 °C, noch bevorzugter 20 bis 30 °C, am bevorzugtesten 35 bis 40 °C betragen.

Zur Anzucht der erfindungsgemäßen Zelle kommen zahlreiche Kulturmedien in Frage, im Falle der Verwendung einer Hefezelle beispielsweise YPD, YPN und YNB, die mit Aminosäuren, beispielsweise mit 0,01 g/L Tryptophan, oder mit Glucose, beispielsweise in einer Konzentration von 1 % (w/v), supplementiert sein können. Im Falle der Verwendung eines Bakteriums aus der Familie der Enterobacteriaceae, bevorzugt *E. coli*, kommen zur Anzucht Vollmedien wie LB-Medium oder Hochzelldichtemedium (HZD-Medium) bestehend aus NH₄SO₄ 1,76 g, K₂HPO₄ 19,08 g, KH₂PO₄ 12,5 g, Hefeextrakt 6,66 g, Na₃-Citrat 1,96 g , NH₄Fe-Citrat (1 %) 17 ml, Spurenelementelösung US3 5 ml, Feedlösung (Glucose 50 % w/v, MgSO₄ [x 7 H₂O 0,5 % w/v, NH₄Cl 2,2 % w/v) 30 ml pro Liter in Frage.

In einer bevorzugten Ausführungsform werden im erfindungsgemäßen Verfahren verwendete Zellen in einem anderen Medium als demjenigen herangezogen, das für die Schritte a) bis d) des Verfahrens verwendet wird. In einer besonders bevorzugten Ausführungsform handelt es sich bei dem für die Anzucht verwendeten Medium um ein Vollmedium und um das für die Schritte a) bis d) verwendete Medium um ein Minimalmedium. Das erfindungsgemäße Verfahren, sofern es unter Verwendung lebensfähiger Zellen durchgeführt wird, wird nach Anzucht der Zellen bevorzugt in Transformationspuffer durchgeführt der pro Liter (NH₄)H₂PO₄ 8 g, NaCl 0,5 g, MgSO₄ x 7 H₂O 0,48 g, Spurenelementlösung US3 15 ml. 1 Liter der Spurenelementelösung US 3 setzte sich zusammen aus HCl 37 % 36,5 g, MnCl₂ x 4H₂O 1,91 g, ZnSO₄ x 7H₂O 1,87 g, Na-EDTA x 2H₂O 0,8 g, H₃BO₃ 0,3 g, Na₂MoO₄ x 2H₂O 0,25 g, CaCl₂ x 2H₂O 4,7 g, FeSO₄ x 7 H₂O 17,8 g, CuCl₂ x 2H₂O 0,15 g umfasst und dessen pH auf 5,4 eingestellt wird.

Die Konzentration von Isobuttersäure, sofern diese zu Beginn des Verfahrens als fertiges Edukt bereitgestellt wird, beträgt zu Beginn der Reaktion 0,01 bis 2, bevorzugter 0,05 bis 1, am bevorzugtesten 0,1 bis 0,2 % (w/v). Wird 3-Hydroxyisobuttersäure unter Verwendung von Glucose als Kohlenstoffquelle hergestellt, die über einen dazu befähigten Stamm zu geeigneten Vorläufern verstoffwechselt wird, so beträgt deren Konzentration zu Beginn im Medium bevorzugt 1 % (w/v).

Die Schritte a) bis d) des erfindungsgemäßen Verfahrens werden bevorzugt bei Atmosphärendruck durchgeführt. Im Falle der Herstellung von Isobuttersäure aus Isobutan kann es vorteilhaft sein, die Alkanhydroxylase die Reaktion in Anwesenheit höherer Drücke in Anwesenheit eines Gasgemisches umfassend, bevorzugt überwiegend umfassend Isobutan. In einer bevorzugten Ausführungsform beträgt der Druck mehr als 1,5, 2, 3 oder 4 bar. In einer weiteren Ausführungsform beträgt der Druck 0,5 bis 4, bevorzugt 1 bis 3, am bevorzugtesten 1 bis 1,5 bar.

In einer bevorzugtesten Ausführungsform der vorliegenden Erfindung wird die der Erfindung zu Grunde liegende Aufgabe dadurch gelöst, dass eine Zelle der Gattung *Yarrowia*, bevorzugt *Yarrowia lipolytica*, bei der die Aktivität der Hydroxyisobuttersäure-Dehydrogenase Yali0F02607g oder einer Variante davon durch Deletion gegenüber der Aktivität der entsprechenden Wildtypzelle gegenüber verringert ist, in wässriger Lösung mit Isobuttersäure kontaktiert wird

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

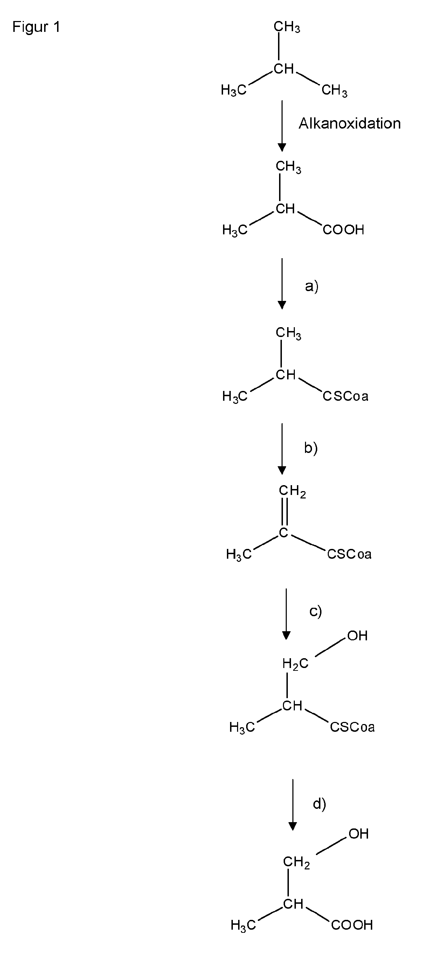

Fig. 1 zeigt eine besonders bevorzugte erfindungsgemäße Sequenz von enzymatisch katalysierten Reaktionen umfassend die der Überführung von Isobuttersäure zu Isobutyryl-CoA durch aus Isobutyrat-Kinase und Phosphotransisobutyrylase oder Isobutyryl-Coenzym A-Synthetase/Ligase oder Isobutyrat-Coenzym A-Transferase in Schritt b), die Oxidation von Isobutyryl-Coenzym A zu Methacrylyl-Coenzym A durch Isobutyryl-Coenzym A-Dehydrogenase in Schritt c), die Addition von Wasser an Methacrylyl-Coenzym A unter Entstehung von 3-Hydroisobutyryl-Coenzym A in Schritt d) und dessen Hydrolyse zu 3-Hydroxyisobuttersäure in Schritt e).

### Beispiele

### Beispiel 1

### Herstellung einer rekombinanten Yarrowia lipolytica-Zelle mit abgeschwächter 3-Hydroxyisobuttersäure-Dehydrogenase-Aktivität

### 1. Konstruktion der Gendisruptionscassette GDC-Yl02607 zur Deletion des 3-Hydroxyisobuttersäure-Dehydrogenase-Gens Yali0F02607g (Accession-Nummer: XP_504911.1)

Zur Konstruktion einer *Yali*0F02607g *knockout*-Mutante in *Y*. *lipolytica* wurde die Promotor- und Terminatorregion des Gens *Yali*0F02607g kloniert. Chromosomale DNA aus *Y. lipolytica* H222 (*MATa*) diente als Matrize für die PCR. Der Gen-*knockout* wurde in den folgenden Stämmen durchgeführt: *Y*. *lipolytica* H222-41 (*MATa ura3-41*) und *Y. lipolytica* H222-SW4-2 (*MATa ura3-302 SUC2 ku70Δ-1572 trp1Δ-1199*). Der Promotor- und Terminatorbereich des für die 3-Hydroxyisobuttersäure-Dehydrogenase (*Yali*0F02607g) kodierenden Gens wurde mit Hilfe der Oligonukleotide 3HIBDH-Pfw (SEQ.-ID-Nr. 01), 3HIBDH-Prv (SEQ.-ID-Nr. 02) (Promotorbereich), 3HIBDH-Tfw (SEQ.-ID-Nr. 03) und 3HIBDH-Trv (SEQ.-ID-Nr. 04) (Terminatorbereich) aus der chromosomalen DNA von *Y. lipolytica* H222 in einer PCR amplifiziert. Folgende Parameter wurden für die PCR eingesetzt: Promotorbereich, 1 x: initiale Denaturierung, 98 °C, 3 min; 35 x: Denaturierung, 98 °C, 0:10 min, Annealing, 59,5 °C, 0:45 min; Elongation, 72 °C, 0:35 min; 1 x: terminale Elongation, 72 °C, 5 min. Terminatorbereich, 1 x: initiale Denaturierung, 98 °C, 3 min; 35 x: Denaturierung, 98 °C, 0:10 min, Annealing, 59,5 °C, 0:45 min; Elongation, 72 °C, 0:35 min; 1 x: terminale Elongation, 72 °C, 5 min. Für die Amplifikation wurde der PhusionTM High-Fidelity Master Mix von New England Biolabs (Frankfurt) entsprechend den Empfehlungen des Herstellers verwendet. Auf diesem Wege wurde das Promotorfragment am 3'-Ende mit einer I-Scel-Schnittstelle und das Terminatorfragment am 5'-Ende mit einer I-Scel-Schnittstelle versehen. Dafür wurden die folgenden Oligonukleotide eingesetzt:
3HIBDH-Pfw:
   5'- CAC ACA TCC AGA GCT CTA TG -3' (SEQ.-ID-Nr. 01)
3HIBDH-Prv:
   5'-TAT ATA CTA TAT TAC CCT GTT ATC CCT AGC GTA ACT ACA AAT ACA AGT TTT AAG CTG -3' (SEQ.-ID-Nr. 02, beinhaltend eine I-Scel-Erkennungssequenz am 5'-Ende)
3HIBDH-Tfw:
   5'- TAT ATA AGT TAC GCT AGG GAT AAC AGG GTA ATA TAG GCT GTG TAT GTG TTA GGG TG - 3' (SEQ.-ID-Nr. 03, beinhaltend eine /-Sce/-Erkennungssequenz am 5'-Ende)
3HIBDH-Trv:
   5'- GGT GAC CTT CAG GTG CAC CA -3' (SEQ.-ID-Nr. 04)

### 2. Fusion des Promotor- und Terminatorfragments

Die PCR-Produkte des Promotor- und Terminatorbereichs (1060 bzw. 970 Basenpaare) wurden mittels des "QIAquick PCR-Purification Kits" (Qiagen, Hilden) gemäß den Angaben des Herstellers aufgereinigt. In einer anschließenden *crossover*-PCR wurden die beiden PCR-Produkte als Matrize eingesetzt und eine Amplifikation mit den Primem 3HIBDH-Pfw (SEQ.-ID-Nr. 01) und 3HIBDH-Trv (SEQ.-ID-Nr. 04) durchgeführt. Folgende Parameter wurden für die PCR eingesetzt: 1 x: initiale Denaturierung, 98 °C, 3 min; 35 x: Denaturierung, 98 °C, 0:10 min, Annealing, 59,5 °C, 0:45 min; Elongation, 72 °C, 1:00 min; 1 x: terminale Elongation, 72 °C, 5 min. Durch die Komplementarität der /-*Sce*/-Restriktionsschnittstelle entstand ein 2,071 Kilobasenpaar großes PCR-Produkt (SEQ.-ID-Nr. 05). Zur Isolierung der DNA aus einem Agarosegel wurde die Ziel-DNA mit einem Skalpell aus dem Gel herausgeschnitten und mit dem "Quick Gel Extraktion Kit" (Qiagen, Hilden) aufgereinigt. Die Durchführung erfolgte nach den Angaben des Herstellers. Im nächsten Schritt wurde das PCR-Produkt in den Vektor pCR-Blunt II-Topo (Zero Blunt TOPO PCR Cloning Kit with One Shot TOP10 Chemically Competent *E. coli*, Invitrogen, Karlsruhe) ligiert. Das resultierende Plasmid pCRBluntIITopo::P_T_3HIBDH_YI (SEQ.-ID-Nr.06) ist 5,59 Kilobasenpaare groß. Die Ligation sowie die Transformation chemisch kompetenter *E. coli* erfolgten nach Angaben des Herstellers. Die Authentizität des Plasmids wurde durch eine Restriktionsanalyse mit E*co*RI, *Xhol* und *Pst*l überprüft.

### 3. Konstruktion der knockout-Mutanten Y. lipolytica H222-SW-4-2 Δ 3HIBDH und Y. lipolytica H222-41 Δ3HIBDH

Zur Konstruktion der *knockout-*Mutante ist die Integration eines Markergens notwendig. Dazu wurde das 1,3 Kilobasenpaare große "IoxP-URA3-IoxR" DNA-Fragment, welches das *URA3*-Gen enthält, mittels einer /-Sce/-Restriktion aus dem Vektor pJMP113 (Fickers *et al.*, 2003) herausgeschnitten. Das "*IoxP-URA3-IoxR"*-Fragment wurde mit Hilfe des "Quick Gel Extraktion Kit" (Qiagen, Hilden) gemäß den Angaben des Herstellers über ein Agarosegel aufgereinigt. Das resultierende Fragment wurde in den mit I-Scel geschnittenen Vektor pCRBIuntIITopo::P_T_3HIBDH_YI (SEQ.-ID-Nr. 06) ligiert. Das resultierende Plasmid pCRBIuntIITopo::P_T_3HIBDH_YI_ura (SEQ.-ID-Nr. 07) ist 6,899 Kilobasenpaare groß. Die Ligation sowie die Transformation chemisch kompetenter *E. coli* DH5α-Zellen (New England Biolabs, Frankfurt) erfolgten in einer dem Fachmann bekannter Art und Weise. Die Authentizität des Plasmids wurde durch eine Restriktion mit *Xma*l, *Sca*l und *Sac*l überprüft.

Um die für den Gen-*knockout* notwendige Gendisruptionscassette GDC-YI02607PT zur Deletion von ORF *Yali*0F02607g zu erhalten, wurde das Plasmid pCRBIuntIITopo::P_T_3HIBDH_YI_ura (SEQ.-ID-Nr. 07) als Matrize für die PCR mit den folgenden Oligonukleotiden und Parametern eingesetzt: 3HIBDH-Pfw (SEQ.-ID-Nr. 01) und 3HIBDH-Trv (SEQ.-ID-Nr. 04); 1 x: initiale Denaturierung, 98 °C, 3 min; 35 x: Denaturierung, 98 °C, 0:10 min, Annealing, 65 °C, 0:45 min; Elongation, 72 °C, 1:45 min; 1 x: terminale Elongation, 72 °C, 5 min. Das gewünschte 3,38 Kilobasenpaare Fragment (SEQ.-ID-Nr. 8) wurde mit Hilfe des "Quick Gel Extraktion Kit" (Qiagen, Hilden) gemäß den Angaben des Herstellers über ein Agarosegel aufgereinigt, überprüft durch die Restriktion mit Xmal und Xmnl und für die integrative Transformation von *Y lipolytica* H222-SW4-2 und *Y lipolytica* H222-41 eingesetzt.

Die Transformation erfolgte mittels der Lithium-Acetat-Methode (Barth G and Gaillardin C (1996) Yarrowia lipolytica. In: Wolf, K. (eds) Nonconventional yeasts in biotechnology. Springer, Berlin Heidelberg New York, pp 313-388). Die erhaltenen Uracil-prototrophen Transformanten wurden mittels Kolonie-PCR überprüft. Folgende Parameter wurden für die PCR eingesetzt: 1 x: initiale Denaturierung, 98 °C, 3 min; 35 x: Denaturierung, 98 °C, 0:10 min, Annealing, 60 °C, 0:45 min; Elongation, 72 °C, 1:30 min; 1 x: terminale Elongation, 72 °C, 5 min. Dazu wurden folgende Oligonukleotide eingesetzt:
fw-3HIBDH-ah:
   5'- GAG TCG CAG ATT CAG GAA AT -3' (SEQ.-ID-Nr. 09)
rv-3HIBDH-ah:
   5'- TCA CCT TCT GAT CAC GGT GT -3' (SEQ.-ID-Nr. 10)

Im Falle einer erfolgreichen Disruption des für die 3-Hydroxyisobuttersäure-Dehydrogenase kodierenden Gens *Yali*0F02607g sollte ein 1,005 Kilobasenpaare großes Fragment amplifiziert werden. In der Tat konnten entsprechende Klone identifiziert werden, die im Folgenden weiter bearbeitet wurden.

### 4. Wiederherstellung der Uracil-Auxotrophie

Die Wiederherstellung der Uracil-Auxotrophie in den Uracil-prototrophen Transformanten erfolgte nach Fickers *et al.,* 2003. Zunächst wurden kompetente Zellen hergestellt. Dazu wurden die Transformanten in 5 mL YPD pH 4 (10 g/L Hefeextrakt, 10 g/L Pepton, 10,5 g/L Zitronensäure, 2 % (w/v) Glucose und 0,5 M Natriumcitrat zur pH-Wert Einstellung) für 8 h bei 30 °C und 190 rpm in 100 mL Schikanenkolben kultiviert. Nach 8 h wurde diese Vorkultur genutzt, um 10 mL YPD-Medium pH 4 in 250 mL-Schikanenkolben mit einer optischen Dichte (OD600) von 0,05 zu inokulieren. Alle Kolben wurden bei 30 °C und 190 rpm inkubiert. Am nächsten Tag bei Erreichen einer Zellzahl zwischen 9·107 und 1,5·107 pro ml (Auszählung mittels der Neubauer-Kammer) wurden diese Vorkulturen geerntet (500 g, 5 min, RT), zweimal in 10 mL TE-Puffer (10 mM Tris-HCl, 1 mM Na-EDTA, pH-Wert 8) gewaschen, anschließend in 30 mL 0,1 % (w/v) Lithium-Acetat verdünnt und 1 h bei 28 °C und 60 rpm inkubiert. Anschließend wurden die Zellen erneut zentrifugiert (500 g, 5 min, RT), in 3 mL 0,1 % (w/v) Lithium-Acetat resuspendiert, in 100 µL aliquotiert und sofort verwendet. Alle weiteren Schritte wurden auf Eis durchgeführt.

Für die Transformation des Plasmids pUB4Cre (Fickers *et al*., 2003) wurden 100 µL kompetente Zellen mit 200 - 800 ng Plasmid-DNA und 2,5 µL aufgekochter Lachsperma-DNA (10 mg/mL) (Invitrogen, Karlsruhe) gemischt. Nach Zugabe von 0,7 mL 40 % PEG4000 (w/v; gelöst in 0,1 M Lithium-Acetat, pH-Wert 6) wurden die Zellen 1 h bei 28 °C unter Schütteln inkubiert. Der Hitzeschock bei 39 °C folgte für 10 min im Wasserbad. Es wurden 1,2 mL 0,1 % (w/v) Lithium-Acetat zu den Zellen gegeben und zweimal 250 µL sehr vorsichtig auf YPD-Platten mit 500 pg/mL Hygromycin aufgetragen.

Nach 3 bis 10 Tagen Inkubation bei 30 °C wurden die erhaltenen Klone phänotypisch auf Agarplatten mit YNB-Medium (6,7 g/L DifcoTM Yeast Nitrogen Base w/o Amino Acids) und Glucose mit oder ohne Uracil überprüft. Um das Plasmid pUB4Cre aus der Zelle zu entfernen, wurden die Tranformanten mehrfach in 10 mL YPD-Medium in 100 mL Schikanenkolben bei 30 °C und 190 rpm für 24 h inkubiert und auf YPD-Platten ausgestrichen. Die resultierenden Klone wurden mittels PCR (wie in Punkt 3 beschrieben) und phänotypisch auf Agarplatten kontrolliert.

Auf diese Weise wurden folgende Stämme konstruiert:
- H222-SW4-2 Δ3HIBDH
- H222-41 Δ3HIBDH

### Beispiel 2

### Produktion von 3-Hydroxyisobuttersäure mit Glucose als alleiniger Kohlenstoffquelle mittels gentechnisch veränderter Y. lipolytica-Zellen, bei denen die Aktivität der 3-Hydroxyiosbuttersäure-Dehydrogenase abgeschwächt wurde

Der in Beispiel 1 konstruierte *Y. lipolytica-*Stamm H222-41 Δ3HIBDH wurden im Vergleich mit dem korrespondierenden Wildtyp H222-41 in 10 mL YNB-Medium (6,7 g/L DifcoTM Yeast Nitrogen Base w/o Amino Acids) mit 0,2 g/L Uracil, 0,01 g/L Tryptophan und 5 % (w/v) Glucose über Nacht bei 28 °C und 190 rpm kultiviert. Am nächsten Morgen wurden diese Vorkulturen genutzt, um je 20 mL YNB-Medium mit 0,2 g/L Uracil, 0,01 g/L Tryptophan und 1 % (w/v) Glucose in 100 mL-Schikanenkolben mit einer optischen Dichte (OD600) von 0,5 zu inokulieren. Alle Kolben wurden bei 28 °C und 190 rpm inkubiert.

Nach einem Zeitraum von 70 h wurde 1 g/L Ammoniumsulfat zugegeben und nach 95 h wurden die Konzentrationen von 3-Hydroxyisobuttersäure in den Ansätzen per IC analysiert. Für die chromatographische Auftrennung in der ICS-2000 RFIC (Dionex, Corporation, Synnyvale, USA) wurde die RFICTM TonPac Säule (2 × 250 mm, Säulentemperatur 30 °C, + Vorsäule AG15 4 × 50 mm, Durchflussrate 0,38 mUmin) verwendet.

Die Stämme erreichten eine OD600 von ca. 30. Während der Kontrollstamm *Y. lipolytica* H222-41 keinerlei 3-Hydroxyisobuttersäure produzierte, konnte im Fall des daraus abgeleiteteten *Y. lipolytica* H222-41 Δ3HIBDH die Bildung von 4,5 mg/L 3-Hydroxyisobuttersäure nachgewiesen werden.

### Beispiel 3

### Produktion von 3-Hydroxyisobuttersäure ausgehend von Isobuttersäure mit gentechnisch veränderten Y. lipolytica-Zellen, bei denen die Aktivität der 3-Hydroxyisobuttersäure-Dehydrogenase abgeschwächt wurde

Die in Beispiel 1 konstruierten *Y. lipolytica*-Stämme H222-SW-4-2 Δ3HIBDH und H222-41 Δ3HIBDH wurden im Vergleich mit einem unmodifizierten Kontroll-Stamm (H222-SW-4-2) in 10 mL YNB-Medium (6,7 g/L DifcoTM Yeast Nitrogen Base w/o Amino Acids) mit 0,01 g/L Tryptophan und 1 % (w/v) Glucose über Nacht bei 30 °C und 190 rpm kultiviert. Am nächsten Morgen wurden diese Vorkulturen genutzt, um je 20 mL YNB-Medium mit 0,2 g/L Uracil, 0,01 g/L Tryptophan, 1 % (w/v) Glucose und 0,2 % (w/v) Isobuttersäure (mit NaOH titriert, pH-Wert: 5,1; nach 24 h wurde erneut 0,2 % Isobuttersäure zugesetzt) in 100 mL-Schikanenkolben mit einer optischen Dichte (OD600) von 0,5 zu inokulieren. Alle Kolben wurden bei 30 °C und 190 rpm inkubiert. Die H222-SW4-2-Stämme erreichten eine OD600 von 2-3 während die H222-41- Stämme bis zu einer OD600 von ca. 10 wuchsen.

Nach einem Zeitraum von 24 h und 48 h wurden die Konzentrationen von 3-Hydroxyisobuttersäure in allen Ansätzen per IC analysiert (siehe Abb. 1). Der Kulturüberstand wurde mit ddH2O 1:10 verdünnt, so dass die Messwerte in dem Kalibrierbereich lagen. Für die chromatographische Auftrennung in der ICS-2000 RFIC (Dionex, Corporation, Synnyvale, USA) wurde die RFICTM TonPac Säule (2 × 250 mm, Säulentemperatur 30 °C, + Vorsäule AG15 4 × 50 mm, Durchflussrate 0,38 mUmin) verwendet.

Nach 24 h erreichten alle *Y. lipolytica*-Stämme die stationäre Wachstumsphase. Der pH-Wert bei den H222-41-Stämmen fiel während des Wachstums auf 3,3 bis 3,7 ab, bei den H222-SW-4-2-Stämmen wurden pH-Werte von 2 detektiert. Nach 24 h und 48 h erreichten die modifizierten *Y. lipolytica*-Stämme H222-SW-4-2 Δ3HIBDH und H222-41 Δ3HIBDH Δ3HIBDH und H222-41 Δ3HIBDH eine höhere Ausbeute als die jeweiligen unmodifizerten Stämme H222-SW-4-2 und H222-41. *Y. lipolytica* H222-41Δ3HIBDH setzt über 90 % Isobuttersäure zu 3-Hydroxyisobuttersäure nach 24 h und über 80 % nach 48 h um.

Durch die Deletion von *Yali*0F02607g in *Y. lipolyica* H222-41 bzw. H222-SW-4-2 konnte die Ausbeute der 3-Hydroxyisobuttersäure-Produktion aus Isobuttersäure deutlich gesteigert werden (siehe Abb. 1).

### Beispiel 4

### Produktion von 3-Hydroxybuttersäure (3-HIB) aus Glucose oder Isobuttersäure (IBA) als alleiniger Kohenstoffquelle durch Y. lipolytica H222 (Wildtyp, WT) und gentechnische veränderten Y. lipolytica H222 Δ3HIBDH (ura)-8 mit abgeschwächter 3-Hydroxyisobuttersäure-Dehydrogenaseaktivität (Δ).

### a) Biomasseproduktion im Schüttelkolbenmaßstab

Kryokulturen von *Y. lipolytica* H222 Δ3HIBDH (ura)-8 (im folgenden bezeichnet als Δ) und *Y. lipolytica* H222 (im folgenden bezeichnet als Wildtyp (WT)) wurden auf YPD-Agarplatten pro Liter hergestellt aus Hefeextrakt 10g, Pepton 20g, Agar-Agar 12g autoklaviert und mit separat autoklavierter Glucose 10g komplementiert, ausgestrichen und für 24h bei 28°C inkubiert.

Pro Stamm wurden jeweils 2 1000 ml-Schüttelkolben mit Schikanen mit 100 ml YPD-Medium (obiges Medium ohne Agar-Agar, mit je 3 Tropfen Antischaum Delamex) gefüllt, mit je 2 vollen Impfösen von den Agarplatten beimpft und für 20h bei 30°C mit 180rpm (Amplitude 2,5cm) inkubiert (Restglucose 0g/l, OD ≥20).

**Tab.1**

| Stamm | pH | OD | Glucose[g/l] |
|---|---|---|---|
| Δ | 7,06 | 20,9 | 0,029 |
| WT | 7,47 | 23,7 | 0,015 |

Die Kulturen wurden dann steril in 50ml Falcons abgefüllt und bei 5000rpm abzentrifugiert. Die Pellets wurden 4x mit 0,9% Saline gewaschen. Danach wurden je 2 Pellets eines Stammes in 50 ml Transformationspuffer resuspendiert und vereinigt.

Der Transformationspuffer setzte sich pro sterilfiltriertem Liter zusammen aus (NH₄)H₂PO₄ 8g, NaCl 0,5g, MgSO₄ x 7 H₂O 0,48 g, Spurenelementlösung US3 15 ml. 1 Liter der Spurenelementelösung US 3 setzte sich zusammen aus HCl 37% 36,5g, MnCl₂ x 4H₂O 1,91g, ZnSO₄ x 7H₂O 1,87g, Na-EDTA x 2H₂O 0,8g, H₃BO₃ 0,3g, Na₂MoO₄ x 2H₂O 0,25g, CaCl₂ x 2H₂O 4,7g, FeSO₄ x 7 H₂O 17,8g, CuCl₂ x 2H₂O 0,15g. Diese Lösung wurde separat sterilfiltriert und steril dem Puffer zugegeben. Der pH des Puffers wurde auf 5,4 eingestellt.

### b) Biotransformation

In 4 1000 ml-Schüttelkolben mit Schikanen wurden steril je 50 ml des genannten Transformationspuffer pH 5,4 mit je 3 Tropfen Antischaum Delamex gegeben. Pro Stamm wurden in einen Schüttelkolben steril für ein Endvolumen von 100ml 0,2% (w/v) Isobuttersäure, in einen zweiten Schüttelkolben 1% (w/v) Glucose gegeben. Je ein Schüttelkolben mit IBA und einer mit Glucose wurde mit je 50 ml resuspendiertem Pelltet von *Y lipolytica* H222 aus a) inokuliert. Ebenso wurde mit *Y. lipolytica* H222 Δ3HIBDH (ura)-8 verfahren. Die Start-OD lag bei ca. 14. Die Schüttelkolben wurden mit 180rpm bei 30°C inkubiert Die Probennahmen erfolgten nach 0, 6 und 24 Stunden. Mikroskopische Kontrollen zeigten über die gesamte Zeit in keinem Ansatz Lysezellen. Die Messung der Glucose erfolgte mit einem YSI Messgerät der Firma Kreienbaum, die Messung von Isobuttersäure und der 3-Hydroxyisobuttersäure erfolgte mittels HPLC über eine Aminex-Säule, die Messung der OD mit einem Spectralphotometer bei 600nm.

Ergebnisse:

**Tab. 2**

| OD | | | | |
|---|---|---|---|---|
| t [h] | WT IBA | WT Glc | Δ IBA | Δ Glc |
| 0 | 13,8 | 13,6 | 15,0 | 13,6 |
| 6 | 13,5 | 19,2 | 12,8 | 25,3 |
| 24 | 13,3 | 18,2 | 10,6 | 16,4 |

Die Glucose wurde zum Teil zum Biomasseaufbau verstoffwechselt. Mit IBA als alleinige C-Quelle fand keine Biomasseproduktion statt.

**Tab. 3**

| Konzentration Substrate IBA / Glucose [mg/l] | | | | | | | | |
|---|---|---|---|---|---|---|---|---|
| Stamm | WT IBA | | WT Glucose | | Δ IBA | | Δ Glucose | |
| t [h] | IBA | Glucose | IBA | Glucose | IBA | Glucose | IBA | Glucose |
| 0 | 1914 | 20 | 0 | 8450 | 1808 | 10 | 0 | 8520 |
| 6 | 676 | 0 | 0 | 3240 | 1267 | 0 | 0 | 1770 |
| 24 | 0 | 0 | 0 | 0 | 422 | 0 | 0 | 0 |

Beide Substrate wurden sowohl von *Y lipolytica* H222, als auch von *Y lipolytica* H222 Δ3HIBDH (ura)-8 verstoffwechselt.

**Tab.4**

| Konzentration 3-HIB [mg/l] | | | | |
|---|---|---|---|---|
| t [h] | WT IBA | WT Glucose | Δ IBA | Δ Glucose |
| 0 | 1 | 1 | 10 | 5 |
| 6 | 50 | 0 | 821 | 21 |
| 24 | 0 | 0 | 2042 | 52 |

Bei *Y lipolytica* H222 konnte nur mit IBA als Substrat 3-HIB kurzzeitig als Stoffwechselprodukt nachgewiesen werden.

Bei Y. lipolytica H222 Δ3HIBDH (ura)-8 konnten mit Glucose als Substrat geringe Mengen freies 3-HIB nachgewiesen werden. Vom eingesetzten IBA wurden 15,7 mmol/l verbraucht. Daraus können theoretisch 18,6 mmol/l 3-HIB entstehen. Gemessen wurden 19,6 mmol/l. Das verbrauchte IBA wird also vollständig zu 3-HIB umgesetzt.

### Beispiel 5

### Produktion von 3-Hydroxyisobuttersäure ausgehend von Ketoisovalerat mit gentechnisch veränderten Y. lipolytica-Zellen, bei denen die Aktivität der 3-Hydroxyisobuttersäure-Dehydrogenase abgeschwächt wurde

Der in Beispiel 1 konstruierte *Y. lipolytica*-Stamm H222-41 Δ3HIBDH wurde im Vergleich mit dem korrespondierenden Wildtypstamm *Y. lipolytica* H222-41 in 10 mL YNB-Medium (6,7 g/L DifcoTM Yeast Nitrogen Base w/o Amino Acids) mit 0,2 g/L Uracil, 0,01 g/L Tryptophan und 1 % (w/v) Glucose über Nacht bei 30 °C und 190 rpm kultiviert. Am nächsten Tag wurden diese Vorkulturen genutzt, um je 25 mL YNB-Medium mit 0,2 g/L Uracil, 0,01 g/L Tryptophan, 6 % (w/v) Glucose und 0,65 % (w/v) Ketoisovalerat (nach 25,5 h wurden erneut 0,5 % (w/v) Ketoisovalerat und nach 49,5 h 0,3 % (w/v) Ketoisovalerat zu der Kultur gegeben) in 100 mL-Schikanenkolben mit einer optischen Dichte (OD600) von 0,5 zu inokulieren. Alle Kolben wurden bei 30 °C und 190 rpm inkubiert.

Über den Verlauf der Zeit wurde die optische Dichte (OD600) bestimmt und der 3-Hydroxyisobuttersäure-Gehalt in allen Ansätzen per IC analysiert. Während der Kulturführung war zu jedem Zeitpunkt ausreichend Glucose und Ketoisovalerat im Medium vorhanden. Nach ca. 24 h erreichten alle *Y. lipolytica*-Stämme die stationäre Wachstumsphase. Der pH-Wert fiel während des Wachstums auf ca. 3 ab.

Durch die Deletion von *Yali*0F02607g in *Y. lipolyica* H222-41 konnte die 3-Hydroxyisobuttersäure-Produktion aus Ketoisovalerat von 2 g/L 3-Hydroxyisobuttersäure auf über 5 g/L 3-Hydroxyisobuttersäure gesteigert werden (siehe Abb. 2).

### Beispiel 6

### Produktion von 3 Hydroxyisobuttersäure mit einem 2 Stufenprozeß ausgehend von iso-Butan mit gentechnisch veränderten E. coli W3110 mit der Monooxygenase (alkBGT) aus P. putida GPO1 zu Isobuttersäure, die von gentechnisch veränderten Y. lipolytica H222 (ura)-8, bei denen die Aktivität der 3-Hydroxyisobuttersäure-Dehydrogenase abgeschwächt wurde, mit IBA als alleiniger Kohlenstoffquelle weiter umgesetzt wird.

### Stufe 1

### Produktion von Isobuttersäure aus Iso-Butan durch E. coli W3110 mit der Monooxygenase (alkBGT) aus P. putida GPO1.

### a) Produktion von Biomasse im 10l Maßstab

Preseedkultur: 1 Liter LB Medium mit 50µl Kanamycin wurde aus einer Lösung von Hefeextrakt 5g, Pepton 10g, NaCl 0,5g und 50µl Kanamycin hergestellt. Der pH wurde mit 5% NH4OH auf 7,4 eingestellt. Die Lösung wird 20 Minuten bei 121 °C autoklaviert.

Von dieser Lösung wurden je 5 x 25ml in 100 ml-Schüttelkolben mit Schikanen gefüllt und mit jeweils 200µl einer Glycerin-Kryokultur von *E. coli* W3110 pBT10 (DE10200710060705) angeimpft. Diese Kulturen wurden für 18 Stunden bei 37°C und 180rpm (Amplitude 2,5cm) inkubiert.

Seedkultur: 1 Liter Hochzelldichtemedium (HZD-Medium) bestehend aus NH₄SO₄ 1,76g, K₂HPO₄ 19,08g, KH₂PO₄ 12,5g, Hefeextrakt 6,66g, Na₃-Citrat 1,96g , NH₄Fe-Citrat (1%) 17ml, Spurenelementelösung US3 5ml, Feedlösung (Glucose 50% w/v, MgSO₄ [x 7 H₂O 0,5% w/v, NH₄Cl 2,2% w/v) 30ml, sowie 50 µg Kanamycin wurde mit VE-Wasser angesetzt. 1 Liter der Spurenelementelösung US 3 setzt sich zusammen aus HCl 37% 36,5g, MnCl₂ x 4H₂O 1,91g, ZnSO₄ x 7H₂O 1,87g, Na-EDTA x 2H₂O 0,8g, H₃BO₃ 0,3g, Na₂MoO₄ x 2H₂O 0,25g, CaCl₂ x 2H₂O 4,7g, FeSO₄ x 7 H₂O 17,8g, CuCl₂ x 2H₂O 0,15g. 948ml Lösung mit NH₄SO₄ bis Na₃-Citrat werden autoklaviert, der Rest wurde jeweils getrennt steril filtriert und im Anschluss steril zugegeben. Der pH lag bei 6,8.

5 x 75 ml des HZD- Mediums in 1000 ml-Schüttelkolben mit Schikanen wurden mit jeweils 25 ml Preseedkultur inokuliert und für 30h bei 37°C und 180rpm (Amplitude 2,5cm) kultiviert. Induktionskultur: Je 25 ml der Kulturbrühe wurden in 75 ml modifiziertes M9-Medium (steril filtriert) mitfolgender Zusammensetzung pro Liter: 15 g Glucose, 6,79 g Na₂PO4, 3 g KH₂PO₄, 0,5 g NaCl, 2 g, NH₄Cl, 15 g Hefeextrakt, 0,49 g MgSO₄*7H₂O, 1 ml Spurenelementlösung (wie in der Seedkultur) und 50 µg Kanamycin in 1000 ml Schüttelkolben überimpft. Die Kulturen werden für 7h bei 35°C und 180rpm (Amplitude 2,5cm) inkubiert.

Ein steriler 10 l-Fermenter wurde mit 7 I eines sterilen Mediums mit der Zusammensetzung (pro Liter) (NH₄)₂SO₄ 1,75 g, K₂HPO₄ x 3 H₂O 19 g, KH₂PO₄ 12,5g, Hefeextrakt 6,6 g, Na₃-Citrat x 2H₂O 2,24 g, Glucose 15 g, MgSO₄ x 7 H₂O 0,49 g, NH₄Fe-Citrat (1% w/v) 16,6ml, Spurenelementlösung (wie in der Seedkultur) 15ml und Kanamycin 50µg und 2 ml Antischaummittel Delamex befüllt. Als Feed wurde eine autoklavierte Lösung von Glucose (50% w/v) mit MgSO₄ x 7H₂O 10g/l angeschlossen, zur pH Korrektur 0,5M H₂SO₄ und 25% NH₄OH.

Die Kulturen aus den Schüttelkolben wurden steril vereinigt und über eine Transferflasche in den Fermenter inokuliert. Als Fermentationsbedingungen wurden eingestellt pO₂ 30%, Airflow 6nlpm, Rührer 400 - 1200rpm Temperatur 37°C, pH7, Feedstart 8 h, Feedrate 150 - 250 g/h. Nach 19 h wurde die Temperatur auf 30°C gesenkt, der Feed gestoppt und mit 0,4 mM DCPK induziert. Nach 23 Stunden beträgt die OD im Fermenter ca. 100, die Kulturbrühe wurde steril entnommen und bei 8000 rpm mit 500 ml in 1000 ml-Zentrifugenbechern abzentrifugiert. Der Überstand wurde verworfen, die Pellets wurden so in sterile Falcons aliquotiert, dass ein resuspendiertes Pellet in 180 ml Trafopuffer eine OD von ca. 20 ergibt. Die Pellets konnten dann sofort in der Biotransformation eingesetzt werden, oder bei -80°C für spätere Verwendung eingefroren werden.

### b) Biotransformation iso-Butan zu Isobuttersäure

Die Pellets aus 200 ml Kultur wurden in 10 ml Konversionspuffer resuspendiert. Der Konversionspuffer bestand aus 70 mM (NH₄)H₂PO₄ Puffer, pH 7 pro Liter mit 8g (NH₄)H₂PO₄, 0,5 g NaCl, 0,49 g MgSO₄ x 7H₂O, 1 ml TE und 50 µg Kanamycin. Die pH-Einstellung erfolgte hier mit 5 % NH₄OH.

150 ml Puffer mit ca. 3 Tropfen autokaviertem Antischaum (Delamex) wurden in einem 300 ml-Fermenter vorgelegt. Der Fermenter wurde mit einem Gasgemisch aus einer Gasflasche mit 5 bar Anfangsdruck von 25 % iso-Butan und 75 % synthetischer Luft über einen Metallsinterperlator mit einer Porengröße von 0,2 µm mit einer Flussrate von 12,5NI/h begast. Der Fermenter wurde in einem Wasserbad auf 35°C temperiert und mit Hilfe eines Magnetrührers mit 900rpm gerührt. Die Abluft wurde durch eine Waschflasche, die mit 150 ml Wasser gefüllt ist, abgeleitet.

Der Fermenter wurde über ein Probenahmerohr mit 30 ml eines in a) hergestellten und in Puffer resuspendierten Pellets angeimpft. Die Reaktion wurde mit dem Start eines Glucosefeedes (oder bei Iso auch Glucosebatch oder Vorlage + Feed....) von 1,5 g/lh begonnen. Nach 4,5 Stunden wurde eine Konzentration von Isobuttersäure von > 350mg/l erreicht (3,97mmol/kg).

Der pH wurde mit 1 M H₂SO₄ auf 5,4 eingestellt und die Begasung auf Druckluft mit einer Flußrate von 12,5NI/h umgestellt, die Temperatur auf 30 Grad gesenkt. (alternativ):
● Aus den Fermentern wurden jeweils 50 ml Transferpufferpuffer mit Kanüle und Spritze steril entnommen zum Resuspendieren der *Yarrowia*kulturen (siehe Stufe 2a)
● die Kultur steril abgeerntet und die Biomasse mittels Zentrifugation bei 8000rpm mit 500ml in 1000ml Zentrifugenbechern abgetrennt, und der Überstand bis zur Transformation mit den *Yarrowia*kulturen bei -20°C aufbewahrt.

### Stufe 2

### Produktion von 3-Hydroxyisobuttersäure aus Isobuttersäure aus Stufe 1 durch gentechnisch veränderte Y. lipolytica H222 Δ3HIBDH (ura)-8 im Vergleich zum Wildtyp Y. lipolytica H222.

### a) Produktion von Biomasse im Schüttelkolbenmaßstab (oder wahlweise auch im Fermenter)

Kryokulturen von *Y. lipolytica* H222 Δ3HIBDH (ura)-8 und *Y. lipolytica* H222 wurden auf YPD-Agarplatten pro Liter hergestellt aus Hefeextrakt 10g, Pepton 20g, Agar-Agar 12g autoklaviert und mit separat autoklavierter Glucose 10g komplementiert, ausgestrichen und für 24h bei 28°C inkubiert. Von den Platten wurden je Stamm jeweils 2 1000 ml-Schüttelkolben mit Schikanen gefüllt mit 100 l YPD-Medium (obiges Medium ohne Agar-Agar, mit je 3 Tropfen Antischaum Delamex) mit je 2 vollen Impfösen angeimpft und für 20h bei 30°C mit 180rpm (Amplitude 2,5cm) inkubiert (Restglucose 0g/l, OD >20).

Die Kulturen wurden dann steril in 50ml Falcons abgefüllt und bei 5000rpm abzentrifugiert. Die Pellets wurden 4x mit 0,9% Saline gewaschen. Danach wurden je 2 Pellets eines Stammes in 50 ml Trafopuffer (aus Stufe 1 b) -frisch oder aufgetaut resuspendiert und vereinigt.

### b) Biotransformation von Isobuttersäure zu 3-Hydroxyisobuttersäure

Je ein mal 50ml der resuspendierten Pellets von *Y. lipolytica* H222 Δ3HIBDH (ura)-8 und *Y. lipolytica* H222 wurden in einem 300ml Fermenter mit dem Trafopuffer aus Stufe 1b) inokuliert und für 24h bei pH 5,4, 30°C und 900rpm gerührt und mit 1,2vvm Druckluft begast.

Nach 24 Stunden konnte im Überstand beim Wildtyp *Y lipolytica* H222 keine Isobuttersäure und keine 3-Hyrdoxyisobuttersäure nachgewiesen werden. Bei *Y lipolytica* H222 Δ3HIBDH (ura)-8 konnte nach 24h im Überstand keine Isobuttersäure mehr nachgewiesen werden. An 3-Hydroxyisobuttersäure wurden entsprechend den am Ende von Stufe 1b) gemessenen Isobuttersäurewerten von > 3,97 mmol Konzentrationen von >413mg/l.

### Literaturstellen:

A. Cornish-Bowden (1995), Fundamentals of Enzym Kinetics, Portland Press Limited, 1995
DE 60216245 (2007): FUNCTIONAL DISPLAY OF POLYPEPTIDES
DE10200710060705 (2007): ω-Aminocarbonsäuren oder ihre Lactame, herstellende, rekombinante Zellen
Sambrook/Fritsch/Maniatis (1989): Molecular Cloning: A Laboratory Manual, Cold Spring Harbor Laboratory Press, 2nd edition
William Bauer, Jr. "Methacrylic Acid and Derivatives" in Ullmann's Encyclopedia of Industrial Chemistry 2002, Wiley-VCH, Weinheim
Hasegawa et al. (1981), J. Ferment. Technol. 59, pp 203-208
Hasegawa (1981 b), Agric. Biol. Chem. 45, pp 2899-2901.
Hasegawa et al. (1982), J. Ferment. Technol. 60, pp 501-508
WO 2007/141208: MICROBIOLOGICAL PRODUCTION OF 3-HYDROXYISOBUTYRIC ACID
WO 2008/119738: ENZYME FOR THE PRODUCTION OF METHYLMALONYL COENZYME A
OR ETHYLMALONYL COENZYME A, AND USE THEREOF
Derilanko et al.(1983), Journal of Applied Biochemistry 5 (1 - 2), 107 - 120)
Barth G and Gaillardin C (1996) Yarrowia lipolytica. In: Wolf, K. (eds) Nonconventional yeasts in biotechnology. Springer, Berlin Heidelberg New York, pp 313-388
Van Beilen et al. (2002): Functional Analysis of Alkane Hydroxylases from Gram-Negative and Gram-Positive Bacteria", Journal of Bacteriology, 2002, 184 (6), pp. 1733-1742).
Fersht A R and Winter G (2008): Redesigning Enzymes by Site-Directed Mutagenesis, Ciba Foundation Symposium 111 - Enzymes in Organic Synthesis
Fuchs/Schlegel (2007) Allgemeine Mikrobiologie, 2008, Georg Thieme Verlag
William Bauer, Jr. "Methacrylic Acid and Derivatives" in Ullmann's Encyclopedia of Industrial Chemistry 2002, Wiley-VCH, Weinheim

## Patentansprüche

1. Verfahren umfassend die folgenden Schritte:
a) Bereitstellen von Isobuttersäure,
b) Kontaktieren von Isobuttersäure mit
der Kombination aus Isobutyrat-Kinase und Phosphotransisobutyrylase und/oder Isobutyryl-Coenzym A-Synthetase/Ligase und/oder
Isobutyrat-Coenzym A-Transferase,
c) Kontaktieren des Produktes aus Schritt b) mit Isobutyryl-Coenzym A-Dehydrogenase,
d) Kontaktieren des Produktes aus Schritt c) mit Methacrylyl-Coenzym A-Hydratase, und
e) Hydrolyse des Produktes aus Schritt d) unter Bildung von 3-Hydroxyisobuttersäure, wobei wenigstens eines der in den Schritten b), c) und d) verwendeten Enzyme aus der Gruppe umfassend Isobutyrat-Kinase, Phosphotransisobutyrylase, Isobutyryl-Coenzym A-Synthetase/Ligase und Isobutyrat-Coenzym A-Transferase, bevorzugt alle, in Form einer Zelle bereitgestellt wird, die eine ihrem Wildtyp gegenüber verringerte Aktivität einer 3-Hydroxyisobuttersäure-Dehydrogenase oder einer Variante davon aufweist.

2. Verfahren nach Anspruch 1, wobei die Isobuttersäure durch Kontaktieren von Isobutan mit einer Monooxygenase, bevorzugt einer Alkanhydroxylase, noch bevorzugter einer des AlkBGT-Typs oder einer Variante davon, gebildet wird.

3. Verfahren nach einem der Ansprüche 1 bis 2, wobei die Hydrolyse in Schritt d) durch Kontaktieren des Produktes aus Schritt d) mit einer 3-Hydroxisobutyryl-Coenzym A-Hydrolase erreicht wird.

4. Verfahren nach einem der Ansprüche 1 bis 3, wobei die Zelle sowohl die Isobutyryl-Coenzym A-Dehydrogenase in Schritt c) als auch die Methacrylyl-Coenzym A-Hydratase in Schritt d) als auch
die Kombination aus Isobutyrat-Kinase und Phosphotransisobutyrylase und/oder Isobutyryl-Coenzym A-Synthetase/Ligase und/oder
Isobutyrat-Coenzym A-Transferase,
aufweist.

5. Verfahren nach einem der Ansprüche 1 bis 4, wobei die Zelle zusätzlich eine Alkanhydroxylase, bevorzugt eine des AlkBGT-Typs oder eine Variante davon, aufweist.

6. Verfahren nach einem der Ansprüche 1 bis 5, wobei es sich bei der 3-Hydroxyisobuttersäure-Dehydrogenase um Yali0F02607g oder eine Variante davon handelt.

7. Zelle, die wenigstens ein Enzym aus der Gruppe umfassend Isobutyryl-Coenzym A-Synthetase/Ligase, Isobutyrat-Coenzym A-Transferase, Isobutyrat-Kinase, Phosphotransisobutyrylase, Isobutyryl-Coenzym A-Dehydrogenase-, Methacrylyl-Coenzym A-Hydratase und 3-Hydroxisobutyryl-Coenzym A-Hydrolase und eine gegenüber ihrem Wildtyp verringerte Aktivität einer 3-Hydroxyisobuttersäure-Dehydrogenase oder einer Variante davon aufweist.

8. Zelle nach Anspruch 7, wobei die Zelle zusätzlich zu einer Isobutyryl-Coenzym A-Dehydrogenase und zusätzlich zu einer Methacrylyl-Coenzym A-Hydratase
die Kombination aus Isobutyrat-Kinase und Phosphotransisobutyrylase und/oder Isobutyryl-Coenzym A-Synthetase/Ligase und/oder
Isobutyrat-Coenzym A-Transferase,
bevorzugt darüber hinaus eine 3-Hydroxisobutyryl-Coenzym A-Hydrolase, aufweist.

9. Zelle nach einem der Ansprüche 7 bis 8, weiter umfassend eine Alkanhydroxylase, bevorzugt eine des AlkBGT-Typs oder eine Variante davon.

10. Zelle nach einem der Ansprüche 6 bis 8, wobei es sich bei der 3-Hydroxyisobuttersäure-Dehydrogenase um Yali0F02607g oder eine Variante davon handelt.

11. Verwendung der Zelle nach einem der Ansprüche 7 bis 10 zur Herstellung von 3-Hydroxyisobuttersäure.

12. Verwendung nach Anspruch 11, wobei es sich bei der 3-Hydroxyisobuttersäure-Dehydrogenase um Yali0F02607g oder eine Variante davon handelt.

13. Verfahren nach einem der Ansprüche 1 bis 6, Zelle nach einem der Ansprüche 7 bis 10 oder Verwendung nach einem der Ansprüche 11 bis 12, wobei es sich bei der Zelle um eine bakterielle oder niedere eukaryontische Zelle handelt.

14. Verfahren nach einem der Ansprüche 1 bis 5 oder 13, Zelle nach einem der Ansprüche 7 bis 10 oder 13 oder Verwendung nach einem der Ansprüche 11 bis 13, wobei es sich um eine Hefezelle aus der Gruppe von Gattungen handelt, die Yarrowia, Candida, Saccharomyces, Schizosaccharomyces und Pichia umfasst und es sich bevorzugt um Yarrowia lipolytica handelt.

15. Reaktionsmischung umfassend die Zelle nach einem der Ansprüche 7 bis 10 oder 13 bis 14 sowie Isobutan oder Isobuttersäure.


SEQUENCE LISTING

<110>  Evonik Degussa GmbH

<120>  Biotechnologische Herstellung von 3-Hydroxyisobuttersäure

<130>  2011DE00275De

<160>  10

<170>  PatentIn version 3.5

<210>  1
<211>  20
<212>  DNA
<213>  Artificial Sequence

<220>
<223>  Primer

<400>  1
cacacatcca gagctctatg                                                    20


<210>  2
<211>  54
<212>  DNA
<213>  Artificial Sequence

<220>
<223>  Primer

<400>  2
atactatatt accctgttat ccctagcgta actacaaata caagttttaa gctg              54


<210>  3
<211>  56
<212>  DNA
<213>  Artificial Sequence

<220>
<223>  Primer

<400>  3
tatataagtt acgctaggga taacagggta atataggctg tgtatgtgtt agggtg            56


<210>  4
<211>  20
<212>  DNA
<213>  Artificial Sequence

<220>
<223>  Primer

<400>  4
ggtgaccttc aggtgcacca                                                    20


<210>  5
<211>  2071
<212>  DNA
<213>  Artificial Sequence

<220>
<223>  PCR product

<400>  5
cacacatcca gagctctatg aagagatggg tatcaaaccc cccaagggag tcattcttta      60

cggcgctccc ggaaccggaa agacactgct ggccaaggcc gtcgccaacc agacttctgc     120

tacatttttg cgaatcgttg gatccgagct gatccaaaag tacttgggag acggtccccg     180

actgtgtcga cagattttcc agattgccgc agaacacgca ccgtccattg ttttcatcga     240

tgagattgac gctattggaa ccaaacgata cgagtcgacg tctggcggag agcgagaggt     300

gcagcggacc atgctggagc ttctgaacca gctggatgga ttcgacgacc gaggagacgt     360

caaggtaatt atggctacaa acaagattga gtcgctggac cctgcactga ttcgacctgg     420

tcgaatcgac cgaaagattc tgtttgagaa tcccgactcc accaccaagc gaaagattat     480

gggcatccac acctccaaga tgaacctcaa cgacgatgtt gatctcgagg agtttgtgtc     540

ttccaaggat gaactttctg gagccgacat caaggccatg tgcaccgagg caggtctgct     600

tgctctgcga gagagacgaa tgcgggtcac tgctgaggat ttccgaaccg ctaaagagcg     660

ggtcatgaag aacaaggtgg aggagaacct tgagggtctc tacctgtaag catagaagca     720

ttaacgtatg actataataa atacatcaat gaggtggagg ttgacgggga cttcttcgta     780

tacatactaa catctaacat ctacagtaga tcgaataact tgcaacggta catgccgtca     840

tagaaagacg tttggtagaa aatgcctaat agagacaaca cacattccaa tacacagaca     900

gttacaacca gcagccacat tgttatataa atgtttacca agtgagccta accagtgggt     960

gtaagtcata catcatcatt cattatttag tcatgatcca tgaaatgttt atgcaatacg    1020

tttcacagtt cattgggtca gcttaaaact tgtatttgtt atataagtta cgctagggat    1080

aacagggtaa tatagtatat agctgtgtat gtgttagggt gggttttggt gtgttggaaa    1140

ggagtgccag aaaagagatc ggtctagctt tatcctctgg cctgcggctc accccagatt    1200

tgaactttgg gtttgtcggg ctagtctcgg ggcgcgtgtg ggggagatgc atcaataacc    1260

gcataatagt ggtcccactg caggtgtttg gaggtggagc tgaaactttt atttcctaat    1320

atcgtattca agctgtctcc aaaggagcat tcatcctcgc aactcctcgg actagctcca    1380

aaaggcattt ttagccgcgt ctgcgtacag gtaagggatc ccccccacat tatccaccca    1440

gcattagtct tgcgttatgt acactcaaca gagttcgtcg attgccgccg aagcggacat    1500

cttcgttggg tgagggacca ggcttgtggt atattaattg gagaggaggg ccgagagggt    1560

tggcgagaag ttacaaattg aactaggagt acacatagaa tgatatacct tctacttcta    1620

ctgacggtcg tagtcacaat acgtgcgtgt gcacaatacg tgcgtgtgta catctgagtt    1680

ataacttgca ctgggttgtc agtgatgaac ccacagtgtg attatgtcct gcccagtgta    1740

caagtacagc actattatgg gtgttgcacc ctatccatct atatacccac catgttagcc    1800

ccgtaagtga gattgattcc cttcatgcgc tccacaagta cagtacatag tcatcggcat    1860

gcatattata tacctacagc atgtacagta tgtactgtaa tagtgggaga tcctacagta 1920

tttgtagtgc tttcttggtg gacatgcata ctttgcacca gtgtcgaaaa tatcgtactt 1980

gtacttaacc actttttgta cttttataac gtgcgtcaaa gcctcttaat tgatgacgct 2040

ccagctatta ttggtgcacc tgaaggtcac c 2071

<210> 6
<211> 5590
<212> DNA
<213> Artificial sequence

<220>
<223> Vektor pCRBluntIITopo::P_T_3HIBDH_Yl

<400> 6
cacacatcca gagctctatg aagagatggg tatcaaaccc cccaagggag tcattcttta 60

cggcgctccc ggaaccggaa agacactgct ggccaaggcc gtcgccaacc agacttctgc 120

tacatttttg cgaatcgttg gatccgagct gatccaaaag tacttgggag acggtccccg 180

actgtgtcga cagattttcc agattgccgc agaacacgca ccgtccattg ttttcatcga 240

tgagattgac gctattggaa ccaaacgata cgagtcgacg tctggcggag agcgagaggt 300

gcagcggacc atgctggagc ttctgaacca gctggatgga ttcgacgacc gaggagacgt 360

caaggtaatt atggctacaa acaagattga gtcgctggac cctgcactga ttcgacctgg 420

tcgaatcgac cgaaagattc tgtttgagaa tcccgactcc accaccaagc gaaagattat 480

gggcatccac acctccaaga tgaacctcaa cgacgatgtt gatctcgagg agtttgtgtc 540

ttccaaggat gaactttctg gagccgacat caaggccatg tgcaccgagg caggtctgct 600

tgctctgcga gagagacgaa tgcgggtcac tgctgaggat ttccgaaccg ctaaagagcg 660

ggtcatgaag aacaaggtgg aggagaacct tgagggtctc tacctgtaag catagaagca 720

ttaacgtatg actataataa atacatcaat gaggtggagg ttgacgggga cttcttcgta 780

tacatactaa catctaacat ctacagtaga tcgaataact tgcaacggta catgccgtca 840

tagaaagacg tttggtagaa aatgcctaat agagacaaca cacattccaa tacacagaca 900

gttacaacca gcagccacat tgttatataa atgtttacca agtgagccta accagtgggt 960

gtaagtcata catcatcatt cattatttag tcatgatcca tgaaatgttt atgcaatacg 1020

tttcacagtt cattgggtca gcttaaaact tgtatttgtt atataagtta cgctagggat 1080

aacagggtaa tatagtatat agctgtgtat gtgttagggt gggttttggt gtgttggaaa 1140

ggagtgccag aaaagagatc ggtctagctt tatcctctgg cctgcggctc accccagatt 1200

tgaactttgg gtttgtcggg ctagtctcgg ggcgcgtgtg ggggagatgc atcaataacc 1260

gcataatagt ggtcccactg caggtgtttg gaggtggagc tgaaactttt atttcctaat 1320

atcgtattca agctgtctcc aaaggagcat tcatcctcgc aactcctcgg actagctcca 1380

aaaggcattt ttagccgcgt ctgcgtacag gtaagggatc ccccccacat tatccaccca 1440

gcattagtct tgcgttatgt acactcaaca gagttcgtcg attgccgccg aagcggacat 1500

cttcgttggg tgagggacca ggcttgtggt atattaattg gagaggaggg ccgagagggt 1560

tggcgagaag ttacaaattg aactaggagt acacatagaa tgatatacct tctacttcta 1620

ctgacggtcg tagtcacaat acgtgcgtgt gcacaatacg tgcgtgtgta catctgagtt 1680

ataacttgca ctgggttgtc agtgatgaac ccacagtgtg attatgtcct gcccagtgta 1740

caagtacagc actattatgg gtgttgcacc ctatccatct atatacccac catgttagcc 1800

ccgtaagtga gattgattcc cttcatgcgc tccacaagta cagtacatag tcatcggcat 1860

gcatattata tacctacagc atgtacagta tgtactgtaa tagtgggaga tcctacagta 1920

tttgtagtgc tttcttggtg gacatgcata ctttgcacca gtgtcgaaaa tatcgtactt 1980

gtacttaacc actttttgta cttttataac gtgcgtcaaa gcctcttaat tgatgacgct 2040

ccagctatta ttggtgcacc tgaaggtcac cagggcgaat tctgcagata tccatcacac 2100

tggcggccgc tcgagcatgc atctagaggg cccaattcgc cctatagtga gtcgtattac 2160

aattcactgg ccgtcgtttt acaacgtcgt gactgggaaa accctggcgt tacccaactt 2220

aatcgccttg cagcacatcc ccctttcgcc agctggcgta atagcgaaga ggcccgcacc 2280

gatcgccctt cccaacagtt gcgcagccta tacgtacggc agtttaaggt ttacacctat 2340

aaaagagaga gccgttatcg tctgtttgtg gatgtacaga gtgatattat tgacacgccg 2400

gggcgacgga tggtgatccc cctggccagt gcacgtctgc tgtcagataa agtctcccgt 2460

gaactttacc cggtggtgca tatcggggat gaaagctggc gcatgatgac caccgatatg 2520

gccagtgtgc cggtctccgt tatcggggaa gaagtggctg atctcagcca ccgcgaaaat 2580

gacatcaaaa acgccattaa cctgatgttc tggggaatat aaatgtcagg catgagatta 2640

tcaaaaagga tcttcaccta gatccttttc acgtagaaag ccagtccgca gaaacggtgc 2700

tgaccccgga tgaatgtcag ctactgggct atctggacaa gggaaaacgc aagcgcaaag 2760

agaaagcagg tagcttgcag tgggcttaca tggcgatagc tagactgggc ggttttatgg 2820

acagcaagcg aaccggaatt gccagctggg gcgccctctg gtaaggttgg gaagccctgc 2880

aaagtaaact ggatggcttt ctcgccgcca aggatctgat ggcgcagggg atcaagctct 2940

gatcaagaga caggatgagg atcgtttcgc atgattgaac aagatggatt gcacgcaggt 3000

tctccggccg cttgggtgga gaggctattc ggctatgact gggcacaaca gacaatcggc 3060

tgctctgatg ccgccgtgtt ccggctgtca gcgcaggggc gcccggttct ttttgtcaag 3120

accgacctgt ccggtgccct gaatgaactg caagacgagg cagcgcggct atcgtggctg 3180

gccacgacgg gcgttccttg cgcagctgtg ctcgacgttg tcactgaagc gggaagggac 3240

tggctgctat tgggcgaagt gccggggcag gatctcctgt catctcacct tgctcctgcc 3300

gagaaagtat ccatcatggc tgatgcaatg cggcggctgc atacgcttga tccggctacc 3360

tgcccattcg accaccaagc gaaacatcgc atcgagcgag cacgtactcg gatggaagcc 3420

ggtcttgtcg atcaggatga tctggacgaa gagcatcagg ggctcgcgcc agccgaactg 3480

ttcgccaggc tcaaggcgag catgcccgac ggcgaggatc tcgtcgtgac ccatggcgat 3540

gcctgcttgc cgaatatcat ggtggaaaat ggccgctttt ctggattcat cgactgtggc 3600

cggctgggtg tggcggaccg ctatcaggac atagcgttgg ctacccgtga tattgctgaa 3660

gagcttggcg gcgaatgggc tgaccgcttc ctcgtgcttt acggtatcgc cgctcccgat 3720

tcgcagcgca tcgccttcta tcgccttctt gacgagttct tctgaattat taacgcttac 3780

aatttcctga tgcggtattt tctccttacg catctgtgcg gtatttcaca ccgcatacag 3840

gtggcacttt tcggggaaat gtgcgcggaa cccctatttg tttatttttc taaatacatt 3900

caaatatgta tccgctcatg agacaataac cctgataaat gcttcaataa tagcacgtga 3960

ggagggccac catggccaag ttgaccagtg ccgttccggt gctcaccgcg cgcgacgtcg 4020

ccggagcggt cgagttctgg accgaccggc tcgggttctc ccgggacttc gtggaggacg 4080

acttcgccgg tgtggtccgg gacgacgtga ccctgttcat cagcgcggtc caggaccagg 4140

tggtgccgga caacaccctg gcctgggtgt gggtgcgcgg cctggacgag ctgtacgccg 4200

agtggtcgga ggtcgtgtcc acgaacttcc gggacgcctc cgggccggcc atgaccgaga 4260

tcggcgagca gccgtggggg cgggagttcg ccctgcgcga cccggccggc aactgcgtgc 4320

acttcgtggc cgaggagcag gactgacacg tgctaaaact tcatttttaa tttaaaagga 4380

tctaggtgaa gatccttttt gataatctca tgaccaaaat cccttaacgt gagttttcgt 4440

tccactgagc gtcagacccc gtagaaaaga tcaaaggatc ttcttgagat cctttttttc 4500

tgcgcgtaat ctgctgcttg caaacaaaaa aaccaccgct accagcggtg gtttgtttgc 4560

cggatcaaga gctaccaact ctttttccga aggtaactgg cttcagcaga gcgcagatac 4620

caaatactgt ccttctagtg tagccgtagt taggccacca cttcaagaac tctgtagcac 4680

cgcctacata cctcgctctg ctaatcctgt taccagtggc tgctgccagt ggcgataagt 4740

cgtgtcttac cgggttggac tcaagacgat agttaccgga taaggcgcag cggtcgggct 4800

gaacgggggg ttcgtgcaca cagcccagct tggagcgaac gacctacacc gaactgagat 4860

acctacagcg tgagctatga gaaagcgcca cgcttcccga agggagaaag gcggacaggt 4920

atccggtaag cggcagggtc ggaacaggag agcgcacgag ggagcttcca gggggaaacg 4980

cctggtatct ttatagtcct gtcgggtttc gccacctctg acttgagcgt cgatttttgt 5040

gatgctcgtc aggggggcgg agcctatgga aaaacgccag caacgcggcc tttttacggt 5100

tcctggcctt ttgctggcct tttgctcaca tgttctttcc tgcgttatcc cctgattctg 5160

tggataaccg tattaccgcc tttgagtgag ctgataccgc tcgccgcagc cgaacgaccg 5220

agcgcagcga gtcagtgagc gaggaagcgg aagagcgccc aatacgcaaa ccgcctctcc 5280

ccgcgcgttg gccgattcat taatgcagct ggcacgacag gtttcccgac tggaaagcgg 5340

gcagtgagcg caacgcaatt aatgtgagtt agctcactca ttaggcaccc caggctttac 5400

actttatgct tccggctcgt atgttgtgtg gaattgtgag cggataacaa tttcacacag 5460

gaaacagcta tgaccatgat tacgccaagc tatttaggtg acactataga atactcaagc 5520

tatgcatcaa gcttggtacc gagctcggat ccactagtaa cggccgccag tgtgctggaa 5580

ttcgccctta 5590

<210> 7
<211> 6899
<212> DNA
<213> Artificial sequence

<220>
<223> pCRBluntIITopo::P_T_3HIBDH_Yl_ura

<400> 7
cacacatcca gagctctatg aagagatggg tatcaaaccc cccaagggag tcattcttta 60

cggcgctccc ggaaccggaa agacactgct ggccaaggcc gtcgccaacc agacttctgc 120

tacatttttg cgaatcgttg gatccgagct gatccaaaag tacttgggag acggtccccg 180

actgtgtcga cagattttcc agattgccgc agaacacgca ccgtccattg ttttcatcga 240

tgagattgac gctattggaa ccaaacgata cgagtcgacg tctggcggag agcgagaggt 300

gcagcggacc atgctggagc ttctgaacca gctggatgga ttcgacgacc gaggagacgt 360

caaggtaatt atggctacaa acaagattga gtcgctggac cctgcactga ttcgacctgg 420

tcgaatcgac cgaaagattc tgtttgagaa tcccgactcc accaccaagc gaaagattat 480

gggcatccac acctccaaga tgaacctcaa cgacgatgtt gatctcgagg agtttgtgtc 540

ttccaaggat gaactttctg gagccgacat caaggccatg tgcaccgagg caggtctgct 600

tgctctgcga gagagacgaa tgcgggtcac tgctgaggat ttccgaaccg ctaaagagcg 660

ggtcatgaag aacaaggtgg aggagaacct tgagggtctc tacctgtaag catagaagca 720

ttaacgtatg actataataa atacatcaat gaggtggagg ttgacgggga cttcttcgta 780

tacatactaa catctaacat ctacagtaga tcgaataact tgcaacggta catgccgtca 840

tagaaagacg tttggtagaa aatgcctaat agagacaaca cacattccaa tacacagaca 900

gttacaacca gcagccacat tgttatataa atgtttacca agtgagccta accagtgggt 960

gtaagtcata catcatcatt cattatttag tcatgatcca tgaaatgttt atgcaatacg 1020

tttcacagtt cattgggtca gcttaaaact tgtatttgtt atataagtta cgctagggat 1080

aacagggtaa tcataacttc gtatagcata cattatacga agttattctg aattccgccc 1140

agagagccat tgacgttctt tctaatttgg accgatagcc gtatagtcca gtctatctat 1200

aagttcaact aactcgtaac tattaccata acatatactt cactgcccca gataaggttc 1260

cgataaaaag ttctgcagac taaatttatt tcagtctcct cttcaccacc aaaatgccct 1320

cctacgaagc tcgagctaac gtccacaagt ccgcctttgc cgctcgagtg ctcaagctcg 1380
tggcagccaa gaaaaccaac ctgtgtgctt ctctggatgt taccaccacc aaggagctca 1440
ttgagcttgc cgataaggtc ggaccttatg tgtgcatgat caagacccat atcgacatca 1500
ttgacgactt cacctacgcc ggcactgtgc tccccctcaa ggaacttgct cttaagcacg 1560
gtttcttcct gttcgaggac agaaagttcg cagatattgg caacactgtc aagcaccagt 1620
accggtgtca ccgaatcgcc gagtggtccg atatcaccaa cgcccacggt gtacccggaa 1680
ccggaatcat tgctggcctg cgagctggtg ccgaggaaac tgtctctgaa cagaagaagg 1740
aggacgtctc tgactacgag aactcccagt acaaggagtt cctagtcccc tctcccaacg 1800
agaagctggc cagaggtctg ctcatgctgg ccgagctgtc ttgcaagggc tctctggcca 1860
ctggcgagta ctccaagcag accattgagc ttgcccgatc cgaccccgag tttgtggttg 1920
gcttcattgc ccagaaccga cctaagggcg actctgagga ctggcttatt ctgacccccg 1980
gggtgggtct tgacgacaag ggagacgctc tcggacagca gtaccgaact gttgaggatg 2040
tcatgtctac cggaacggat atcataattg tcggccgagg tctgtacggc cagaaccgag 2100
atcctattga ggaggccaag cgataccaga aggctggctg ggaggcttac cagaagatta 2160
actgttagag gttagactat ggatatgtaa tttaactgtg tatatagaga gcgtgcaagt 2220
atggagcgct gttcagcttg tatgatggtc agacgacctg tctgatcgag tatgtatggt 2280
actgcacaac ctgtgtatcc gcatggtctg tccaatgggg catgttgttg tgtttctcgg 2340
aattcgtata acttcgtata gcaggagtta tccgaagcga tatagggata acagggtaat 2400
atagtatata gctgtgtatg tgttagggtg ggttttggtg tgttggaaag gagtgccaga 2460
aaagagatcg gtctagcttt atcctctggc ctgcggctca ccccagattt gaactttggg 2520
tttgtcgggc tagtctcggg gcgcgtgtgg gggagatgca tcaataaccg cataatagtg 2580
gtcccactgc aggtgtttgg aggtggagct gaaactttta tttcctaata tcgtattcaa 2640
gctgtctcca aaggagcatt catcctcgca actcctcgga ctagctccaa aaggcatttt 2700
tagccgcgtc tgcgtacagg taaggatccc cccccacatt atccacccag cattagtctt 2760
gcgttatgta cactcaacag agttcgtcga ttgccgccga agcggacatc ttcgttgggt 2820
gagggaccag gcttgtggta tattaattgg agaggagggc cgagagggtt ggcgagaagt 2880
tacaaattga actaggagta cacatagaat gatatacctt ctacttctac tgacggtcgt 2940
agtcacaata cgtgcgtgtg cacaatacgt gcgtgtgtac atctgagtta taacttgcac 3000
tgggttgtca gtgatgaacc cacagtgtga ttatgtcctg cccagtgtac aagtacagca 3060
ctattatggg tgttgcaccc tatccatcta tatacccacc atgttagccc cgtaagtgag 3120
attgattccc ttcatgcgct ccacaagtac agtacatagt catcggcatg catattatat 3180
acctacagca tgtacagtat gtactgtaat agtgggagat cctacagtat ttgtagtgct 3240

ttcttggtgg acatgcatac tttgcaccag tgtcgaaaat atcgtacttg tacttaacca 3300

ctttttgtac ttttataacg tgcgtcaaag cctcttaatt gatgacgctc cagctattat 3360

tggtgcacct gaaggtcacc agggcgaatt ctgcagatat ccatcacact ggcggccgct 3420

cgagcatgca tctagagggc ccaattcgcc ctatagtgag tcgtattaca attcactggc 3480

cgtcgtttta caacgtcgtg actgggaaaa ccctggcgtt acccaactta atcgccttgc 3540

agcacatccc cctttcgcca gctggcgtaa tagcgaagag gcccgcaccg atcgcccttc 3600

ccaacagttg cgcagcctat acgtacggca gtttaaggtt tacacctata aaagagagag 3660

ccgttatcgt ctgtttgtgg atgtacagag tgatattatt gacacgccgg ggcgacggat 3720

ggtgatcccc ctggccagtg cacgtctgct gtcagataaa gtctcccgtg aactttaccc 3780

ggtggtgcat atcggggatg aaagctggcg catgatgacc accgatatgg ccagtgtgcc 3840

ggtctccgtt atcggggaag aagtggctga tctcagccac cgcgaaaatg acatcaaaaa 3900

cgccattaac ctgatgttct ggggaatata aatgtcaggc atgagattat caaaaaggat 3960

cttcacctag atccttttca cgtagaaagc cagtccgcag aaacggtgct gaccccggat 4020

gaatgtcagc tactgggcta tctggacaag ggaaaacgca agcgcaaaga gaaagcaggt 4080

agcttgcagt gggcttacat ggcgatagct agactgggcg gttttatgga cagcaagcga 4140

accggaattg ccagctgggg cgccctctgg taaggttggg aagccctgca aagtaaactg 4200

gatggctttc tcgccgccaa ggatctgatg gcgcagggga tcaagctctg atcaagagac 4260

aggatgagga tcgtttcgca tgattgaaca agatggattg cacgcaggtt ctccggccgc 4320

ttgggtggag aggctattcg gctatgactg ggcacaacag acaatcggct gctctgatgc 4380

cgccgtgttc cggctgtcag cgcaggggcg cccggttctt tttgtcaaga ccgacctgtc 4440

cggtgccctg aatgaactgc aagacgaggc agcgcggcta tcgtggctgg ccacgacggg 4500

cgttccttgc gcagctgtgc tcgacgttgt cactgaagcg ggaagggact ggctgctatt 4560

gggcgaagtg ccggggcagg atctcctgtc atctcacctt gctcctgccg agaaagtatc 4620

catcatggct gatgcaatgc ggcggctgca tacgcttgat ccggctacct gcccattcga 4680

ccaccaagcg aaacatcgca tcgagcgagc acgtactcgg atggaagccg gtcttgtcga 4740

tcaggatgat ctggacgaag agcatcaggg gctcgcgcca gccgaactgt tcgccaggct 4800

caaggcgagc atgcccgacg gcgaggatct cgtcgtgacc catggcgatg cctgcttgcc 4860

gaatatcatg gtggaaaatg gccgcttttc tggattcatc gactgtggcc ggctgggtgt 4920

ggcggaccgc tatcaggaca tagcgttggc tacccgtgat attgctgaag agcttggcgg 4980

cgaatgggct gaccgcttcc tcgtgcttta cggtatcgcc gctcccgatt cgcagcgcat 5040

cgccttctat cgccttcttg acgagttctt ctgaattatt aacgcttaca atttcctgat 5100

gcggtatttt ctccttacgc atctgtgcgg tatttcacac cgcatacagg tggcactttt 5160

cggggaaatg tgcgcggaac ccctatttgt ttatttttct aaatacattc aaatatgtat 5220

ccgctcatga gacaataacc ctgataaatg cttcaataat agcacgtgag gagggccacc 5280

atggccaagt tgaccagtgc cgttccggtg ctcaccgcgc gcgacgtcgc cggagcggtc 5340

gagttctgga ccgaccggct cgggttctcc cgggacttcg tggaggacga cttcgccggt 5400

gtggtccggg acgacgtgac cctgttcatc agcgcggtcc aggaccaggt ggtgccggac 5460

aacaccctgg cctgggtgtg ggtgcgcggc ctggacgagc tgtacgccga gtggtcggag 5520

gtcgtgtcca cgaacttccg ggacgcctcc gggccggcca tgaccgagat cggcgagcag 5580

ccgtgggggc gggagttcgc cctgcgcgac ccggccggca actgcgtgca cttcgtggcc 5640

gaggagcagg actgacacgt gctaaaactt catttttaat ttaaaaggat ctaggtgaag 5700

atcctttttg ataatctcat gaccaaaatc ccttaacgtg agttttcgtt ccactgagcg 5760

tcagaccccg tagaaaagat caaaggatct tcttgagatc ctttttttct gcgcgtaatc 5820

tgctgcttgc aaacaaaaaa accaccgcta ccagcggtgg tttgtttgcc ggatcaagag 5880

ctaccaactc tttttccgaa ggtaactggc ttcagcagag cgcagatacc aaatactgtc 5940

cttctagtgt agccgtagtt aggccaccac ttcaagaact ctgtagcacc gcctacatac 6000

ctcgctctgc taatcctgtt accagtggct gctgccagtg gcgataagtc gtgtcttacc 6060

gggttggact caagacgata gttaccggat aaggcgcagc ggtcgggctg aacggggggt 6120

tcgtgcacac agcccagctt ggagcgaacg acctacaccg aactgagata cctacagcgt 6180

gagctatgag aaagcgccac gcttcccgaa gggagaaagg cggacaggta tccggtaagc 6240

ggcagggtcg gaacaggaga gcgcacgagg gagcttccag ggggaaacgc ctggtatctt 6300

tatagtcctg tcgggtttcg ccacctctga cttgagcgtc gatttttgtg atgctcgtca 6360

ggggggcgga gcctatggaa aaacgccagc aacgcggcct ttttacggtt cctggccttt 6420

tgctggcctt ttgctcacat gttctttcct gcgttatccc ctgattctgt ggataaccgt 6480

attaccgcct ttgagtgagc tgataccgct cgccgcagcc gaacgaccga gcgcagcgag 6540

tcagtgagcg aggaagcgga agagcgccca atacgcaaac cgcctctccc cgcgcgttgg 6600

ccgattcatt aatgcagctg gcacgacagg tttcccgact ggaaagcggg cagtgagcgc 6660

aacgcaatta atgtgagtta gctcactcat taggcacccc aggctttaca ctttatgctt 6720

ccggctcgta tgttgtgtgg aattgtgagc ggataacaat ttcacacagg aaacagctat 6780

gaccatgatt acgccaagct atttaggtga cactatagaa tactcaagct atgcatcaag 6840

cttggtaccg agctcggatc cactagtaac ggccgccagt gtgctggaat tcgccctta 6899

<210> 8
<211> 3380
<212> DNA
<213> Artificial sequence

<220>
<223> PCR product II

<400> 8
cacacatcca gagctctatg aagagatggg tatcaaaccc cccaagggag tcattcttta 60
cggcgctccc ggaaccggaa agacactgct ggccaaggcc gtcgccaacc agacttctgc 120
tacatttttg cgaatcgttg gatccgagct gatccaaaag tacttgggag acggtccccg 180
actgtgtcga cagattttcc agattgccgc agaacacgca ccgtccattg ttttcatcga 240
tgagattgac gctattggaa ccaaacgata cgagtcgacg tctggcggag agcgagaggt 300
gcagcggacc atgctggagc ttctgaacca gctggatgga ttcgacgacc gaggagacgt 360
caaggtaatt atggctacaa acaagattga gtcgctggac cctgcactga ttcgacctgg 420
tcgaatcgac cgaaagattc tgtttgagaa tcccgactcc accaccaagc gaaagattat 480
gggcatccac acctccaaga tgaacctcaa cgacgatgtt gatctcgagg agtttgtgtc 540
ttccaaggat gaactttctg gagccgacat caaggccatg tgcaccgagg caggtctgct 600
tgctctgcga gagagacgaa tgcgggtcac tgctgaggat ttccgaaccg ctaaagagcg 660
ggtcatgaag aacaaggtgg aggagaacct tgagggtctc tacctgtaag catagaagca 720
ttaacgtatg actataataa atacatcaat gaggtggagg ttgacgggga cttcttcgta 780
tacatactaa catctaacat ctacagtaga tcgaataact tgcaacggta catgccgtca 840
tagaaagacg tttggtagaa aatgcctaat agagacaaca cacattccaa tacacagaca 900
gttacaacca gcagccacat tgttatataa atgtttacca agtgagccta accagtgggt 960
gtaagtcata catcatcatt cattatttag tcatgatcca tgaaatgttt atgcaatacg 1020
tttcacagtt cattgggtca gcttaaaact tgtatttgtt atataagtta cgctagggat 1080
aacagggtaa tcataacttc gtatagcata cattatacga agttattctg aattccgccc 1140
agagagccat tgacgttctt tctaatttgg accgatagcc gtatagtcca gtctatctat 1200
aagttcaact aactcgtaac tattaccata acatatactt cactgcccca gataaggttc 1260
cgataaaaag ttctgcagac taaatttatt tcagtctcct cttcaccacc aaaatgccct 1320
cctacgaagc tcgagctaac gtccacaagt ccgcctttgc cgctcgagtg ctcaagctcg 1380
tggcagccaa gaaaaccaac ctgtgtgctt ctctggatgt taccaccacc aaggagctca 1440
ttgagcttgc cgataaggtc ggaccttatg tgtgcatgat caagacccat atcgacatca 1500
ttgacgactt cacctacgcc ggcactgtgc tccccctcaa ggaacttgct cttaagcacg 1560
gtttcttcct gttcgaggac agaaagttcg cagatattgg caacactgtc aagcaccagt 1620
accggtgtca ccgaatcgcc gagtggtccg atatcaccaa cgcccacggt gtacccggaa 1680
ccggaatcat tgctggcctg cgagctggtg ccgaggaaac tgtctctgaa cagaagaagg 1740
aggacgtctc tgactacgag aactcccagt acaaggagtt cctagtcccc tctcccaacg 1800
agaagctggc cagaggtctg ctcatgctgg ccgagctgtc ttgcaagggc tctctggcca 1860
ctggcgagta ctccaagcag accattgagc ttgcccgatc cgaccccgag tttgtggttg 1920

gcttcattgc ccagaaccga cctaagggcg actctgagga ctggcttatt ctgacccccg 1980

gggtgggtct tgacgacaag ggagacgctc tcggacagca gtaccgaact gttgaggatg 2040

tcatgtctac cggaacggat atcataattg tcggccgagg tctgtacggc cagaaccgag 2100

atcctattga ggaggccaag cgataccaga aggctggctg ggaggcttac cagaagatta 2160

actgttagag gttagactat ggatatgtaa tttaactgtg tatatagaga gcgtgcaagt 2220

atggagcgct gttcagcttg tatgatggtc agacgacctg tctgatcgag tatgtatggt 2280

actgcacaac ctgtgtatcc gcatggtctg tccaatgggg catgttgttg tgtttctcgg 2340

aattcgtata acttcgtata gcaggagtta tccgaagcga tatagggata acagggtaat 2400

atagtatata gctgtgtatg tgttagggtg ggttttggtg tgttggaaag gagtgccaga 2460

aaagagatcg gtctagcttt atcctctggc ctgcggctca ccccagattt gaactttggg 2520

tttgtcgggc tagtctcggg gcgcgtgtgg gggagatgca tcaataaccg cataatagtg 2580

gtcccactgc aggtgtttgg aggtggagct gaaactttta tttcctaata tcgtattcaa 2640

gctgtctcca aaggagcatt catcctcgca actcctcgga ctagctccaa aaggcatttt 2700

tagccgcgtc tgcgtacagg taagggatcc cccccacatt atccacccag cattagtctt 2760

gcgttatgta cactcaacag agttcgtcga ttgccgccga agcggacatc ttcgttgggt 2820

gagggaccag gcttgtggta tattaattgg agaggagggc cgagagggtt ggcgagaagt 2880

tacaaattga actaggagta cacatagaat gatatacctt ctacttctac tgacggtcgt 2940

agtcacaata cgtgcgtgtg cacaatacgt gcgtgtgtac atctgagtta taacttgcac 3000

tgggttgtca gtgatgaacc cacagtgtga ttatgtcctg cccagtgtac aagtacagca 3060

ctattatggg tgttgcaccc tatccatcta tatacccacc atgttagccc cgtaagtgag 3120

attgattccc ttcatgcgct ccacaagtac agtacatagt catcggcatg catattatat 3180

acctacagca tgtacagtat gtactgtaat agtgggagat cctacagtat ttgtagtgct 3240

ttcttggtgg acatgcatac tttgcaccag tgtcgaaaat atcgtacttg tacttaacca 3300

ctttttgtac ttttataacg tgcgtcaaag cctcttaatt gatgacgctc cagctattat 3360

tggtgcacct gaaggtcacc 3380

<210> 9
<211> 20
<212> DNA
<213> Artificial sequence

<220>
<223> Primer

<400> 9
gagtcgcaga ttcaggaaat 20

<210> 10

<211>  20
<212>  DNA
<213>  Artificial sequence

<220>
<223>  Primer

<400>  10
tcaccttctg atcacggtgt                                                    20